# EUROPEAN PATENT SPECIFICATION

(11) **EP 0 668 762 B1**
(45) Date of publication and mention of the grant of the patent: **17.09.2003**
(21) Application number: 94902223.0
(22) Date of filing: 01.11.1993
(51) Int. Cl.: A61K 31/335, C07D 305/14, C07D 205/08, A61P 35/02

(54) **ISOBUTENYL-SUBSTITUTED TAXANES AND THEIR PHARMACEUTICAL COMPOSITIONS**
ISOBUTENYL-SUBSTITUIERTE TAXANE UND DEREN PHARMAZEUTISCHE ZUSAMMENSETZUNGEN
TAXANES A SUBSTITUTION ISOBUTENYLE ET COMPOSITIONS PHARMACEUTIQUES LES CONTENANT

(30) Priority: 13.11.1992 US 975705; 15.01.1993 US 5229; 29.01.1993 US 10798; 05.03.1993 US 26978; 22.03.1993 US 34247; 20.07.1993 US 94719
(43) Date of publication of application: 30.08.1995
(60) Divisional application: 02019283.7
(73) Proprietor: FLORIDA STATE UNIVERSITY, Tallahassee, Florida 32310 (US)
(72) Inventor: HOLTON, Robert, A. Innovation Park, Tallahassee, FL 32306-2763 (US); CHAI, Ki-Byung Innovation Park, Tallahassee, FL 32306-2763 (US); NADIZADEH, Hossain Innovation Park, Tallahassee, FL 32306-2763 (US)
(74) Representative: Eyles, Christopher Thomas
(86) International application number: US9310813
(87) International publication number: WO94010996

(56) References cited:
- EP-A- 0 400 971
- US-A- 4 680 391
- US-A- 5 175 315

## Description

### BACKGROUND OF THE INVENTION

The present invention is directed to novel taxanes which have utility as antileukemia and antitumor agents.

The taxane family of terpenes, of which taxol is a member, has attracted considerable interest in both the biological and chemical arts. Taxol is a promising cancer chemotherapeutic agent with a broad spectrum of antileukemic and tumor-inhibiting activity. Taxol has a 2'R, 3'S configuration and the following structural formula: 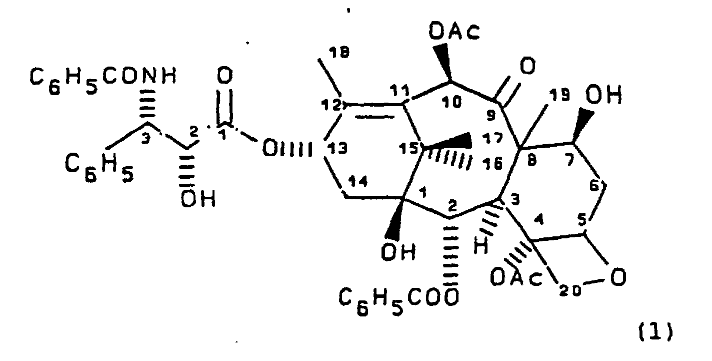 wherein Ac is acetyl. Because of this promising activity, taxol is currently undergoing clinical trials in both France and the United States.

Colin et al. reported in U.S. Patent No. 4,814,470 that taxol derivatives having structural formula (**2**) below, have an activity significantly greater than that of taxol (**1**). 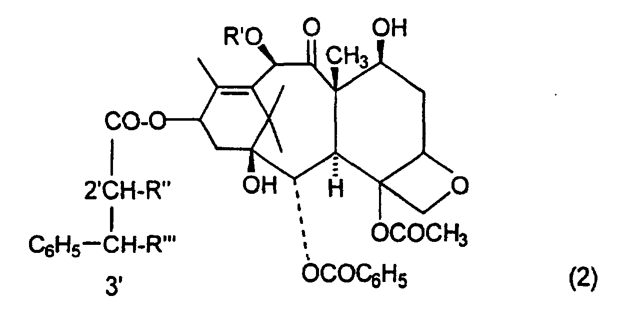 R' represents hydrogen or acetyl and one of R" and R''' represents hydroxy and the other represents tert-butoxycarbonylamino and their stereoisomeric forms, and mixtures thereof. The compound of formula (2) in which R' is hydrogen, R" is hydroxy, R"' is tert-butoxycarbonylamino having the 2'R, 3'S configuration is commonly referred to as taxotere.

Although taxol and taxotere are promising chemotherapeutic agents, they are not universally effective. Accordingly, a need remains for additional chemotherapeutic agents.

In European Patent Publication No. 0400971 A2 there are disclosed certain β-lactams and a process for the preparation of taxol which comprises contacting the β-lactam and an alcohol in the presence of an activating agent to provide a taxol intermediate which is then converted to taxol. Such β-lactams have the formula: 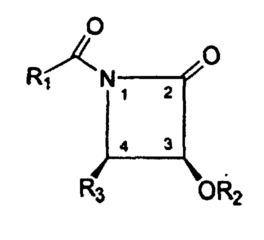 wherein R₁ is aryl, substituted aryl, alkenyl, or alkynyl; R₂ is hydrogen, ethoxyethyl, acetal or other hydroxyl protecting group; and R₃ is aryl, substituted aryl, alkyl, alkenyl, or alkynyl.

In U.S. Patent No. 4,680,391 Firestone et al. disclose certain azetidinones which are said to be potent elastase inhibitors and hence useful anti-inflammatory/anti-degenerative agents.

### SUMMARY OF THE INVENTION

Among the objects of the present invention, therefore, is the provision of novel taxane derivatives which are valuable antileukemia and antitumor agents.

Briefly, therefore, the present invention is directed to taxane derivatives having a C13 side chain which includes an isobutenyl substituent and corresponds to the formula: 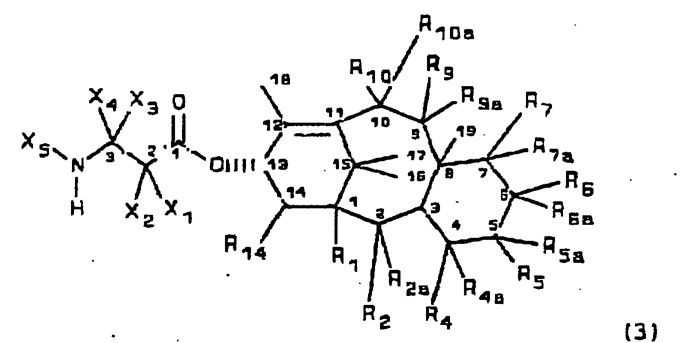 wherein
X₁ is -OX₆, -SX₇, or -NX₈X₉;
X₂ is hydrogen, alkyl, alkenyl, alkynyl, aryl, or heteroaryl;
X₃ is hydrogen;
X₄ is isobutenyl;
X₅ is -COX₁₀, -COOX₁₀, -COSX₁₀, -CONX₈X₁₀, or -SO₂X₁₁;
X₆ is hydrogen, alkyl, alkenyl, alkynyl, aryl, or heteroaryl;
X₇ is alkyl, alkenyl, alkynyl, aryl, heteroaryl, or a sulfhydryl protecting group;
X₈ is hydrogen, alkyl, alkenyl, alkynyl, aryl, heteroaryl, or heterosubstituted alkyl, alkenyl, alkynyl, aryl or heteroaryl;
X₉ is an amino protecting group;
X₁₀ is alkyl, alkenyl, alkynyl, aryl, heteroaryl, or heterosubstituted alkyl, alkenyl, alkynyl, aryl or heteroaryl;
X₁₁ is alkyl, alkenyl, alkynyl, aryl, heteroaryl, -OX₁₀, or -NX₈X₁₄;
X₁₄ is hydrogen, alkyl, alkenyl, alkynyl, aryl, or heteroaryl;
R₁ is hydrogen, hydroxy, protected hydroxy or together with R₁₄ forms a carbonate;
R₂ is hydrogen, hydroxy, -OCOR₃₁ or together with R₂ₐ forms an oxo;
R₂ₐ is hydrogen or taken together with R₂ forms an oxo;
R₄ is hydrogen, together with R₄ₐ forms an oxo, oxirane or methylene, or together with R₅ₐ and the carbon atoms to which they are attached form an oxetane ring;
R₄ₐ is hydrogen, alkyl, alkenyl, alkynyl, aryl, heteroaryl, cyano, hydroxy, -OCOR₃₀, or together with R₄ forms an oxo, oxirane or methylene;
R₅ is hydrogen or together with R₅ₐ forms an oxo,
R₅ₐ is hydrogen, hydroxy, protected hydroxy, acyloxy, together with R₅ forms an oxo, or together with R₄ and the carbon atoms to which they are attached form an oxetane ring;
R₆ is hydrogen, alkyl, alkenyl, alkynyl, aryl, or heteroaryl, hydroxy, protected hydroxy or together with R₆ₐ forms an oxo;
R₆ₐ is hydrogen, alkyl, alkenyl, alkynyl, aryl, or heteroaryl, hydroxy, protected hydroxy or together with R₆ forms an oxo;
R₇ is hydrogen or together with R₇ₐ forms an oxo,
R₇ₐ is hydrogen, halogen, protected hydroxy, -OR₂₈, or together with R₇ forms an oxo;
R₉ is hydrogen or together with R₉ₐ forms an oxo,
R₉ₐ is hydrogen, hydroxy, protected hydroxy, acyloxy, or together with R₉ forms an oxo;
R₁₀ is hydrogen or together with R₁₀ₐ forms an oxo,
R₁₀ₐ is hydrogen, -OCOR₂₉, hydroxy, or protected hydroxy, or together with R₁₀ forms an oxo;
R₁₄ is hydrogen, alkyl, alkenyl, alkynyl, aryl, heteroaryl, hydroxy, protected hydroxy or together with R₁ forms a carbonate;
R₁₄ₐ is hydrogen, alkyl, alkenyl, alkynyl, aryl, or heteroaryl;
R₂₈ is hydrogen, acyl, or a hydroxy protecting group; and
R₂₉, R₃₀, and R₃₁ are independently hydrogen, alkyl, alkenyl, alkynyl, monocyclic aryl or monocyclic heteroaryl.

Other objects and features of this invention will be in part apparent and in part pointed out hereinafter.

### DETAILED DESCRIPTION OF THE PREFERRED EMBODIMENTS

As used herein "Ar" means aryl; "Ph" means phenyl; "Ac" means acetyl; "Et" means ethyl; "R" means alkyl unless otherwise defined; "Bu" means butyl; "Pr" means propyl; "TES" means triethylsilyl; "TMS" means trimethylsilyl; "TPAP" means tetrapropylammonium perruthenate; "DMAP" means p-dimethylamino pyridine; "DMF" means dimethylformamide; "LDA" means lithium diisopropylamide; "LHMDS" means lithium hexamethyldisilazide; "LAH" means lithium aluminum hydride; "Red-Al" means sodium bis(2-methoxyethoxy) aluminum hydride; "AIBN" means azo-(bis)-isobutyronitrile; "10-DAB" means 10-desacetylbaccatin III; FAR means 2-chloro-1,1,2-trifluorotriethylamine; "protected hydroxy" means -OR wherein R is a hydroxy protecting group; "sulfhydryl protecting group" includes, but is not limited to, hemithioacetals such as 1-ethoxyethyl and methoxymethyl, thioesters, or thiocarbonates; "amine protecting group" includes, but is not limited to, carbamates, for example, 2,2,2-trichloroethylcarbamate or tertbutylcarbamate; and "hydroxy protecting group" includes, but is not limited to, ethers such as methyl, t-butyl, benzyl, p-methoxybenzyl, p-nitrobenzyl, allyl, trityl, methoxymethyl, 2-methoxypropyl, methoxyethoxymethyl, ethoxyethyl, tetrahydropyranyl, tetrahydrothiopyranyl, and trialkylsilyl ethers such as trimethylsilyl ether, triethylsilyl ether, dimethylarylsilyl ether, triisopropylsilyl ether and t-butyldimethylsilyl ether; esters such as benzoyl, acetyl, phenylacetyl, formyl, mono-, di-, and trihaloacetyl such as chloroacetyl, dichloroacetyl, trichloroacetyl, trifluoroacetyl; and carbonates including but not limited to alkyl carbonates having from one to six carbon atoms such as methyl, ethyl, n-propyl, isopropyl, n-butyl, t-butyl; isobutyl, and n-pentyl; alkyl carbonates having from one to six carbon atoms and substituted with one or more halogen atoms such as 2,2,2-trichloroethoxymethyl and 2,2,2-trichloroethyl; alkenyl carbonates having from two to six carbon atoms such as vinyl and allyl; cycloalkyl carbonates having from three to six carbon atoms such as cyclopropyl, cyclobutyl, cyclopentyl and cyclohexyl; and phenyl or benzyl carbonates optionally substituted on the ring with one or more C₁₋₆ alkoxy, or nitro. Other hydroxyl, sulfhydryl and amine protecting groups may be found in "Protective Groups in Organic Synthesis" by T. W. Greene, John Wiley and Sons, 1981.

The alkyl groups described herein, either alone or with the various substituents defined herein are preferably lower alkyl containing from one to six carbon atoms in the principal chain and up to 15 carbon atoms. They may be substituted, straight, branched chain or cyclic and include methyl, ethyl, propyl, isopropyl, butyl, hexyl, cyclopropyl, cyclopentyl, and cyclohexyl.

The alkenyl groups described herein, either alone or with the various substituents defined herein are preferably lower alkenyl containing from two to six carbon atoms in the principal chain and up to 15 carbon atoms. They may be substituted, straight or branched chain and include ethenyl, propenyl, isopropenyl, butenyl, isobutenyl, and hexenyl.

The alkynyl groups described herein, either alone or with the various substituents defined herein are preferably lower alkynyl containing from two to six carbon atoms in the principal chain and up to 15 carbon atoms. They may be substituted, straight or branched chain and include ethynyl, propynyl, butynyl, isobutynyl, and hexynyl.

The aryl moieties described herein, either alone or with various substituents, contain from 6 to 15 carbon atoms and include phenyl. Substituents include alkanoxy, protected hydroxy, halogen, alkyl, aryl, alkenyl, acyl, acyloxy, nitro, amino, and amido. Phenyl is the more preferred aryl.

The heteroaryl moieties described herein, either alone or with various substituents, contain from 5 to 15 atoms and include furyl, thienyl, pyridyl and the like. Substituents include alkanoxy, protected hydroxy, halogen, alkyl, aryl, alkenyl, acyl, acyloxy, nitro, amino, and amido.

The acyloxy groups described herein contain alkyl, alkenyl, alkynyl, aryl or heteroaryl groups.

The substituents of the substituted alkyl, alkenyl, alkynyl, aryl, and heteroaryl groups and moieties described herein, may be alkyl, alkenyl, alkynyl, aryl, heteroaryl and/or may contain nitrogen, oxygen, sulfur, halogens and include, for example, lower alkoxy such as methoxy, ethoxy, butoxy, halogen such as chloro or fluoro, nitro, amino, and keto.

In accordance with the present invention, it has been discovered that compounds corresponding to structural formula **3** show remarkable properties, in vitro, and are valuable antileukemia and antitumor agents. Their biological activity has been determined in vitro, using tubulin assays according to the method of Parness et al., J. Cell Biology, 91: 479-487 (1981) and human cancer cell lines, and is comparable to that exhibited by taxol and taxotere.

In one embodiment of the present invention, the substituents of the cyclic nucleus of the taxane (other than the C13 substituent) correspond to the substituents present on baccatin III or 10-DAB. That is, R₁₄ and R₁₄ₐ are hydrogen, R₁₀ is hydrogen, R₁₀ₐ is hydroxy or acetoxy, R₉ and R₉ₐ together form an oxo, R₇ is hydrogen, R₇ₐ is hydroxy, R₅ is hydrogen, R₅ₐ and R₄ and the carbons to which they are attached form an oxetane ring, R₄ₐ is acetoxy, R₂ is hydrogen, R₂ₐ is bensoyloxy, and R₁ is hydroxy. In other embodiments, the taxane has a structure which differs from that of taxol or taxotere with respect to the C13 side chain and at least one other substituent. For example, R₁₄ may be hydroxy, R₂ may be hydroxy or -OCOR₃₁ wherein R₃₁ is hydrogen, alkyl or selected from the group consisting comprising 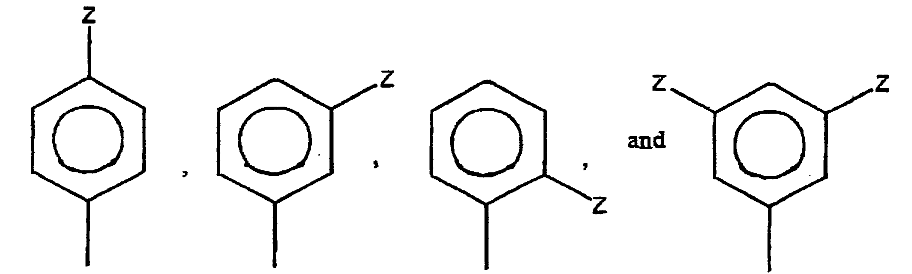 and Z is alkyl, hydroxy, alkoxy, halogen, or trifluoromethyl. R₉ₐ may be hydrogen and R₉ may be hydrogen or hydroxy, R₇ₐ may be hydrogen and R₇ may be acetoxy or other acyloxy or halogen, or R₁₀ and R₁₀ₐ may each be hydrogen or together form an oxo.

With respect to the C13 side chain, in a preferred embodiment X₁ is -OH, X₂ is hydrogen, X₃ is hydrogen, X₄ is isobutenyl, X₅ is -COX₁₀ wherein X₁₀ is furyl, thienyl, alkyl substituted furyl or thienyl, or X₅ is -COOX₁₀ wherein X₁₀ is t-butyl, isobutyl, or n-butyl, ethyl, isopropyl, n-propyl, cyclohexyl, allyl, crotyl, 1,3-diethoxy-2-propyl, 2-methoxyethyl, amyl, neopentyl, or PhCH₂-, or X₅ is -NX₈X₁₀ wherein X₈ and X₁₀ are both phenyl, or X₈ is hydrogen and X₁₀ is n-propyl, phenyl or ethyl.

Taxanes having the general formula **3** may be obtained by reacting a β-lactam with alkoxides having the taxane tricyclic or tetracyclic nucleus and a C-13 metallic oxide substituent to form compounds having a β-amido ester substituent at C-13. The β-lactams have the following structural formula: 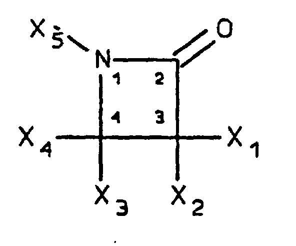 wherein X₁ - X₅ are as defined above.

The β-lactams can be prepared from readily available materials; as is illustrated in schemes A and B below: 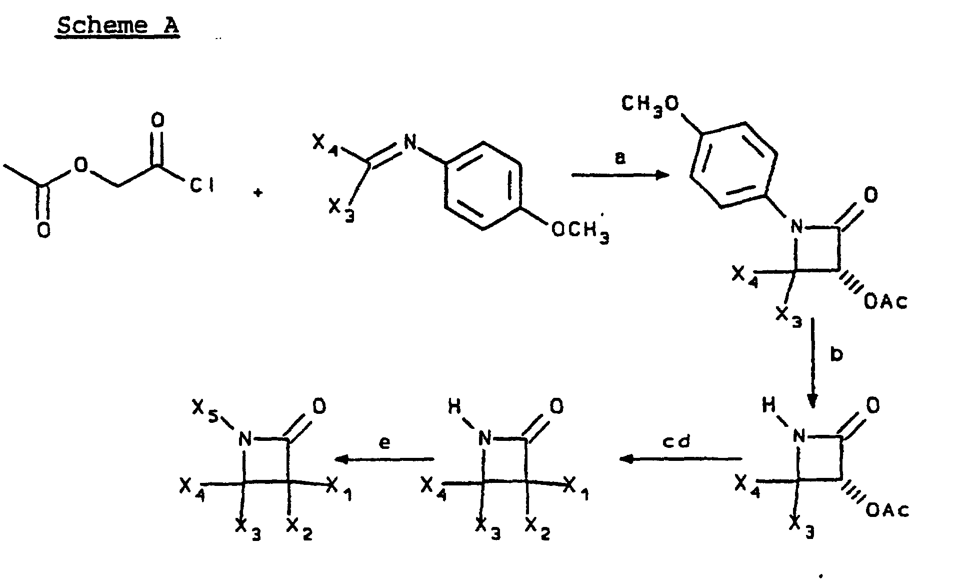 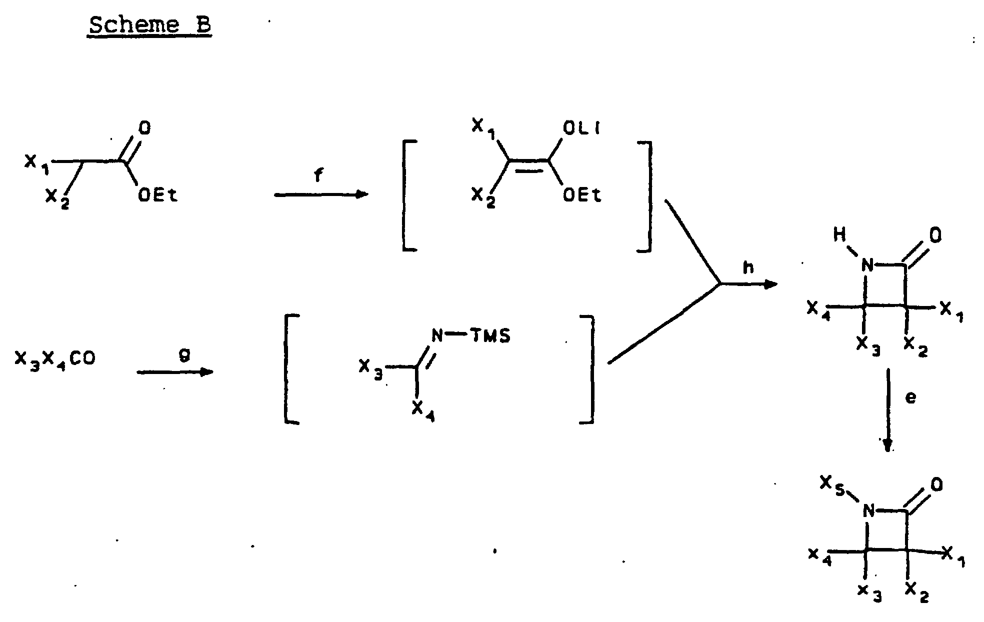 reagents: (a) triethylamine, CH₂Cl₂, 25°C, 18h; (b) 4 equiv ceric ammonium nitrate, CH₃CN, -10°C, 10 min; (c) KOH, THF, H₂O, 0°C, 30 min, or pyrolidine, pyridine, 25 °C, 3h, (d) TESCl, pyridine, 25 °C, 30 min or 2-methoxypropene toluene sulfonic acid (cat.), THF, 0°C, 2h; (e) n-butyllithium, THF, -78 °C, 30 min; and an acyl chloride or chloroformate (X₅ = -COX₁₀), sulfonyl chloride (X₅ = -COSX₁₀) or isocyanate (X₅ = -CONX₈X₁₀); (f) lithium diisopropyl amide, THF -78°C to -50°C; (g) lithium hexamethyldisilazide, THF -78°C to 0°C; (h) THF, -78°C to 25°C, 12h.

The starting materials are readily available. In scheme A, α-acecoxy acetyl chloride is prepared from glycolic acid, and, in the presence of a tertiary amine, it cyclocondenses with imines prepared from aldehydes and p-methoxyaniline to give 1-p-methoxyphenyl-3-acyloxy-4-arylazetidin-2-ones. The p-methoxyphenyl group can be readily removed through oxidation with ceric ammonium nitrate, and the acyloxy group can be hydrolyzed under standard conditions familiar to those experienced in the art to provide 3-hydroxy-4-arylazetidin-2-ones. In Scheme B, ethyl-α-triethylsilyloxyacetate is readily prepared from glycolic acid.

In Schemes A and B, X₁ is preferably -OX₆ and X₆ is a hydroxy protecting group. Protecting groups such as 2-methoxypropyl ("MOP"), 1-ethoxyethyl ("EE") are preferred, but a variety of other standard protecting groups such as the triethylsilyl group or other trialkyl (or aryl) silyl groups may be used. As noted above, additional hydroxy protecting groups and the synthesis thereof may be found in "Protective groups in Organic Synthesis" by T.W. Greene, John Wiley & Sons, 1981.

The racemic β-lactams may be resolved into the pure enantiomers prior to protection by recrystallization of the corresponding 2-methoxy-2-(trifluoromethyl) phenylacetic esters. However, the reaction described hereinbelow in which the β-amido ester side chain is attached has the advantage of being highly diastereoselective, thus permitting the use of a racemic mixture of side chain precursor.

The alkoxides having the tricyclic or tetracyclic taxane nucleus and a C-13 metallic oxide or ammonium oxide substituent have the following structural formula: 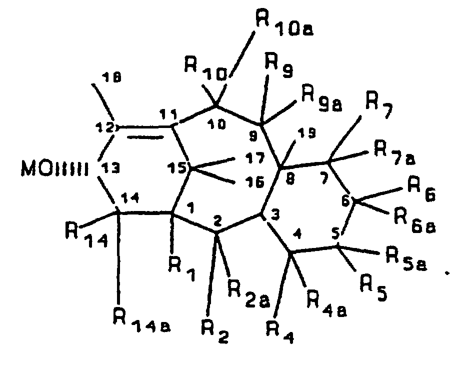 wherein R₁ - R₁₄ₐ are as previously defined and M comprises ammonium or is a metal optionally selected from the group comprising Group IA, Group IIA and transition metals, and preferably, Li, Mg, Na, K or Ti. Most preferably, the alkoxide has the tetracyclic taxane nucleus and corresponds to the structural formula: 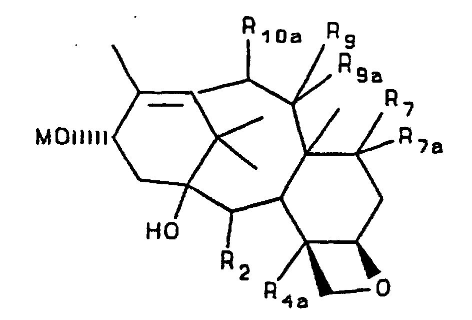 wherein M, R₂, R₄ₐ, R₇, R₇ₐ, R₉, R₉ₐ, R₁₀, and R₁₀ₐ are as previously defined.

The alkoxides can be prepared by reacting an alcohol having the taxane nucleus and a C-13 hydroxyl group with an organometallic compound in a suitable solvent. Most preferably, the alcohol is a protected baccatin III, in particular, 7-O-trïethylsilyl baccatin III (which can be obtained as described by Greene, et al. in JACS 110: 5917 (1988) or by other routes) or 7,10-bis-O-triethylsilyl baccatin III.

As reported in Greene et al., 10-deacetyl baccatin III is converted to 7-O-triethylsilyl-10-deacetyl baccatin III according to the following reaction scheme: 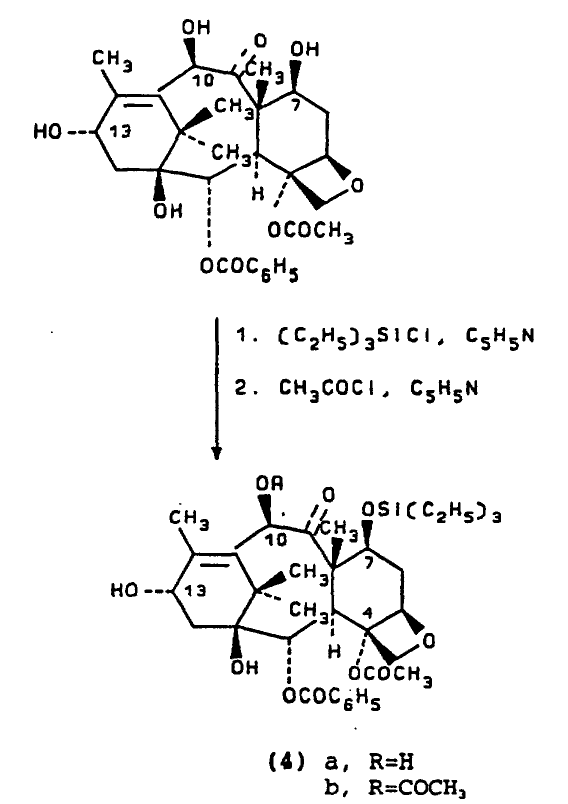 Under what is reported to be carefully optimized conditions, 10-deacetyl baccatin III is reacted with 20 equivalents of (C₂H₅)₃SiCl at 23°C under an argon atmosphere for 20 hours in the presence of 50 ml of pyridine/mmol of 10-deacetyl baccatin III to provide 7-triethylsilyl-10-deacetyl baccatin III **(4a)** as a reaction product in 84-86% yield after purification. The reaction product may then optionally be acetylated with 5 equivalents of CH₃COCl and 25 mL of pyridine/mmol of **4a** at 0 °C under an argon atmosphere for 48 hours to provide 86% yield of 7-O-triethylsilyl baccatin III (**4b**). Greene, et al. in JACS 110, 5917 at 5918 (1988).

The 7-protected baccatin III (**4b**) is reacted with an organometallic compound such as LHMDS in a solvent such as tetrahydrofuran (THF), to form the metal alkoxide 13-O-lithium-7-O-triethylsilyl baccatin III as shown in the following reaction scheme:

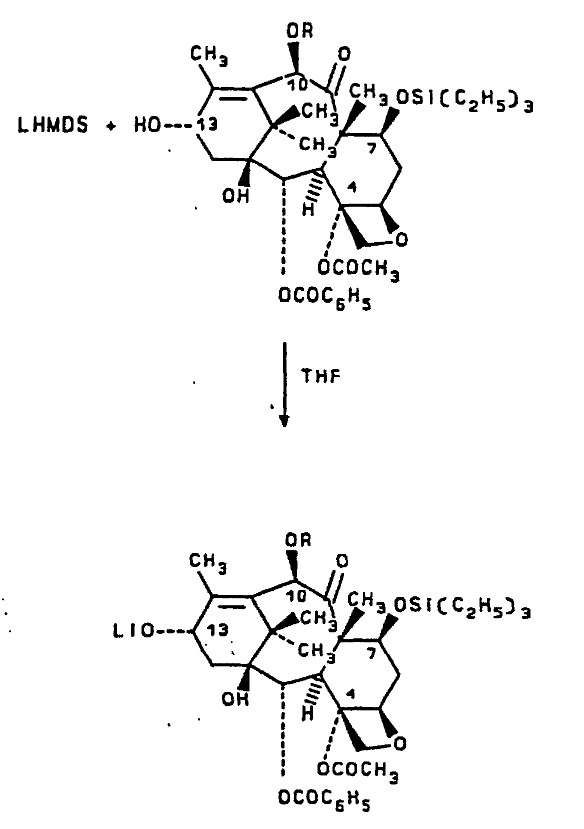

As shown in the following reaction scheme, 13-O-lithium-7-O-triethylsilyl baccatin III reacts with a β-lactam in which X₁ is preferably -OX₆, (X₆ being a hydroxy protecting group) and X₂ - X₅ are as previously defined to provide an intermediate in which the C-7 and C-2' hydroxyl groups are protected. The protecting groups are then hydrolyzed under mild conditions so as not to disturb the ester linkage or the taxane substituents.

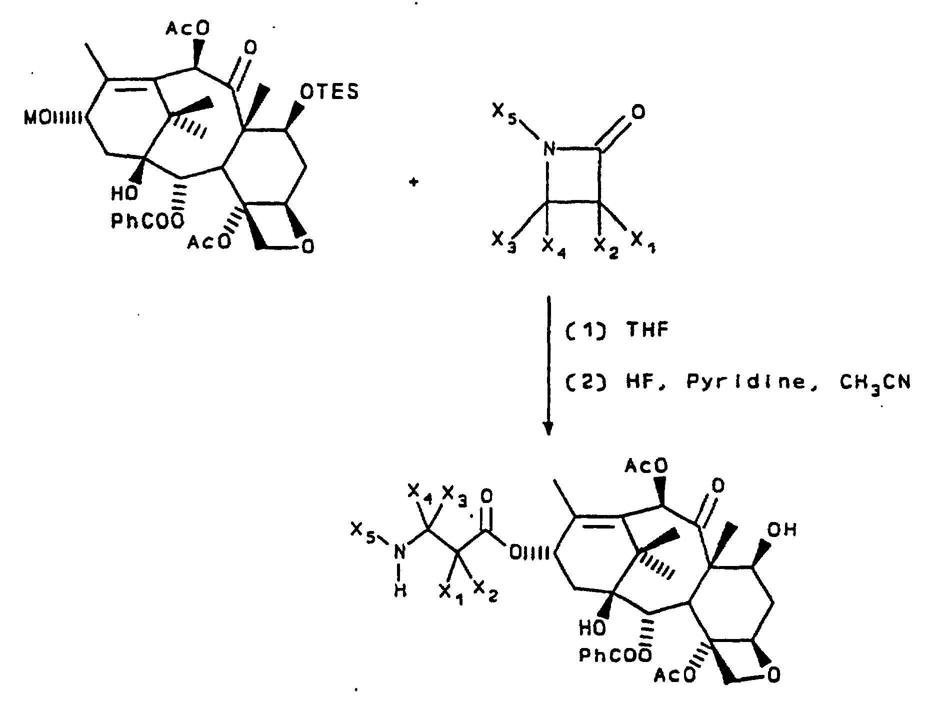

Both the conversion of the alcohol to the alkoxide and the ultimate synthesis of the taxane derivative can take place in the same reaction vessel. Preferably, the β-lactam is added to the reaction vessel after formation therein of the alkoxide.

Compounds of formula 3 of the instant invention are useful for inhibiting tumor growth in animals including humans and are preferably administered in the form of a pharmaceutical composition comprising an effective antitumor amount of compound of the instant invention in combination with a pharmaceutically acceptable carrier or diluent.

Antitumor compositions herein may be made up in any suitable form appropriate for desired use; e.g., oral, parenteral or topical administration. Examples of parenteral administration are intramuscular, intravenous, intraperitoneal, rectal and subcutaneous administration.

The diluent or carrier ingredients should not be such as to diminish the therapeutic effects of the antitumor compounds.

Suitable dosage forms for oral use include tablets, dispersible powders, granules, capsules, suspensions, syrups, and elixirs. Inert diluents and carriers for tablets include, for example, calcium carbonate, sodium carbonate, lactose and talc. Tablets may also contain granulating and disintegrating agents such as starch and alginic acid, binding agents such as starch, gelatin and acacia, and lubricating agents such as magnesium stearate, stearic acid and talc. Tablets may be uncoated or may be coated by unknown techniques; e.g.-, to delay disintegration and absorption. Inert diluents and carriers which may be used in capsules include, for example, calcium carbonate, calcium phosphate and kaolin. Suspensions, syrups and elixirs may contain conventional excipients, for example, methyl cellulose, tragacanth, sodium alginate; wetting agents, such as lecithin and polyoxyethylene stearate; and preservatives, e.g., ethyl- p-hydroxybenzoate.

Dosage forms suitable for parenteral administration include solutions, suspensions, dispersions, emulsions and the like. They may also be manufactured in the form of sterile solid compositions which can be dissolved or suspended in sterile injectable medium immediately before use. They may contain suspending or dispersing agents known in the art.

Taxanes having alternative C9 keto substituent may be prepared by selectively reducing the C₉ keto substituent to yield the corresponding C9 β-hydroxy derivative. The reducing agent is preferably a borohydride and, most preferably, tetrabutylammoniumboro-hydride (Bu₄NBH₄) or triacetoxyborohydride.

As illustrated in Reaction Scheme 1, the reaction of baccatin III with Bu₄NBH₄ in methylene chloride yields 9-desoxo-9β-hydroxybaccatin III **5**. After the C7 hydroxy group is protected with the triethylsilyl protecting group, for example, a suitable side chain may be attached to 7-protected-9β-hydroxy derivative **6** as elsewhere described herein. Removal of the remaining protecting groups thus yields 9β-hydroxy-desoxo taxol or other 9β-hydroxytetracyclic taxane having a C13 side chain.

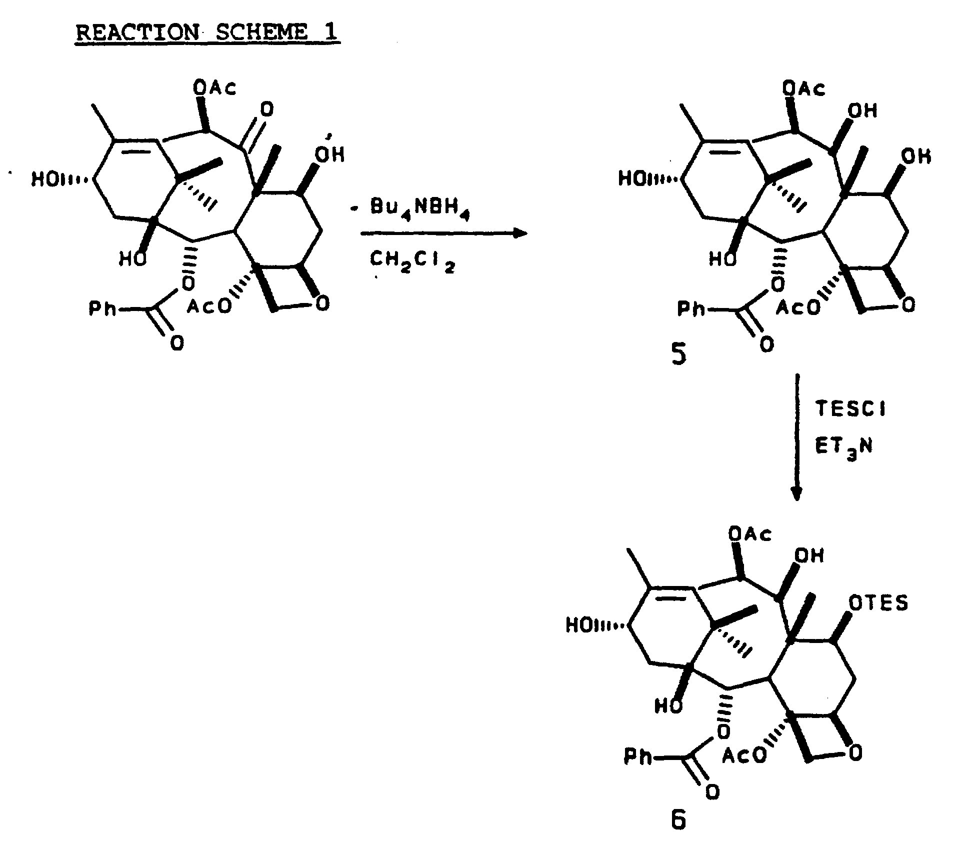

Alternatively, the C13 hydroxy group of 7-protected-9β-hydroxy derivative **6** may be protected with trimethylsilyl or other protecting group which can be selectively removed relative to the C7 hydroxy protecting group as illustrated in Reaction Scheme 2, to enable further selective manipulation of the various substituents of the taxane. For example, reaction of 7,13-protected-9β-hydroxy derivative **7** with KH causes the acetate group to migrate from C10 to C9 and the hydroxy group to migrate from C9 to C10, thereby yielding 10-desacetyl derivative **8**. Protection of the C10 hydroxy group of 10-desacetyl derivative 8 with triethylsilyl yields derivative **9**. Selective removal of the C13 hydroxy protecting group from derivative **9** yields derivative **10** to which a suitable side chain may be attached as described above.

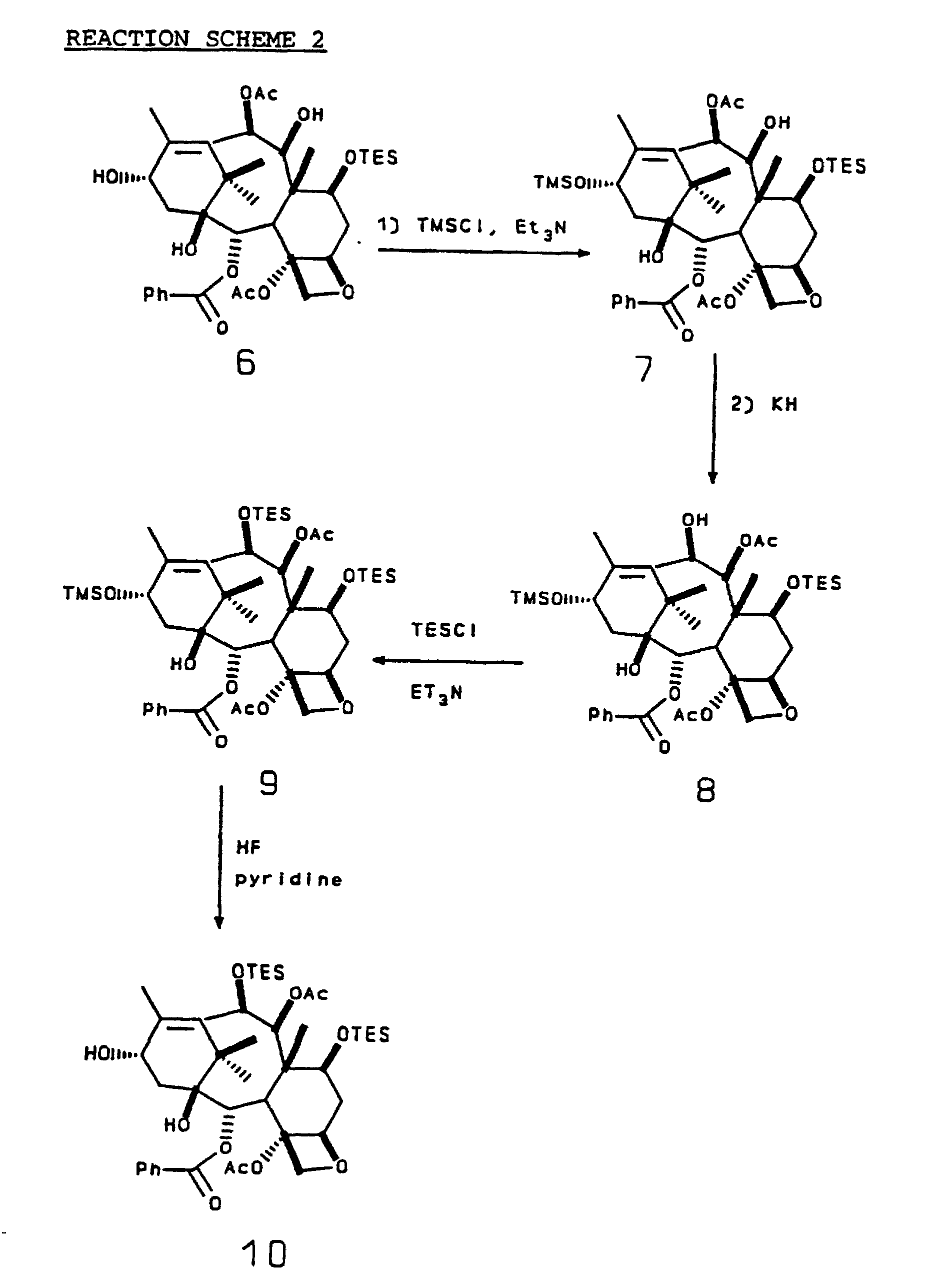

As shown in Reaction Scheme 3, 10-oxo derivative **11** can be provided by oxidation of 10-desacetyl derivative **8**. Thereafter, the C13 hydroxy protecting group can be selectively removed followed by attachment of a side chain as described above to yield 9-acetoxy-10-oxo-taxol or other 9-acetoxy-10-oxotetracyclic taxanes having a C13 side chain. Alternatively, the C9 acetate group can be selectively removed by reduction of 10-oxo derivative **11** with a reducing agent such as samarium diiodide to yield 9-desoxo-10-oxo derivative **12** from which the C13 hydroxy protecting group can be selectively removed followed by attachment of a side chain as described above to yield 9-desoxo-10-oxo-taxol or other 9-desoxo-10-oxotetracyclic taxanes having a C13 side chain.

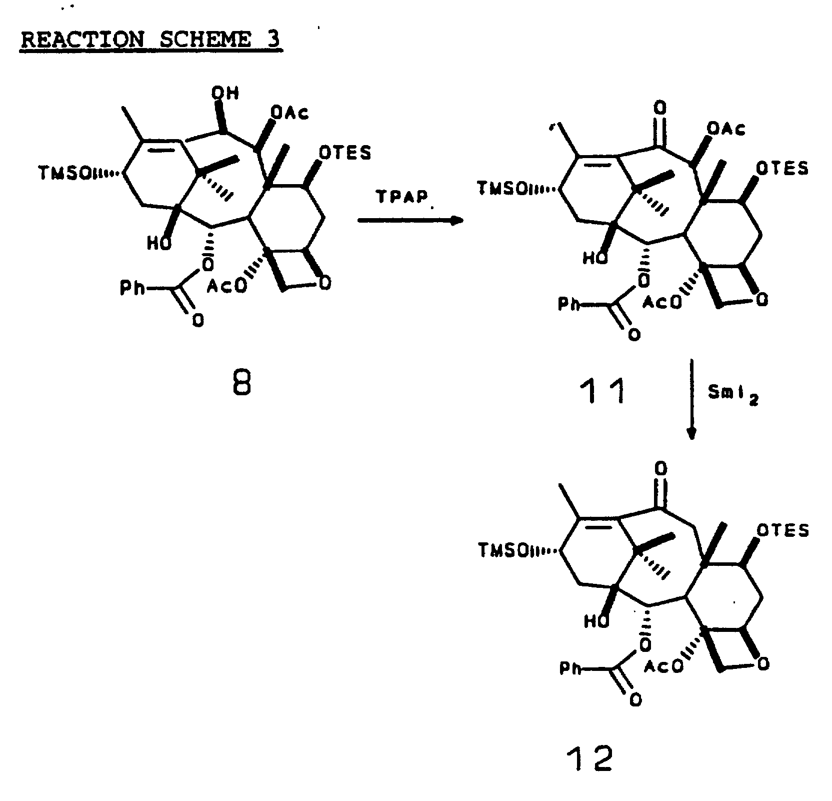

Reaction Scheme 4 illustrates a reaction in which 10-DAB is reduced to yield pentaol **13.** The C7 and C10 hydroxyl groups of pentaol **13** can then be selectively protected with the triethylsilyl or another protecting group to produce triol **14** to which a C13 side chain can be attached as described above or, alternatively, after further modification of the tetracyclic substituents.

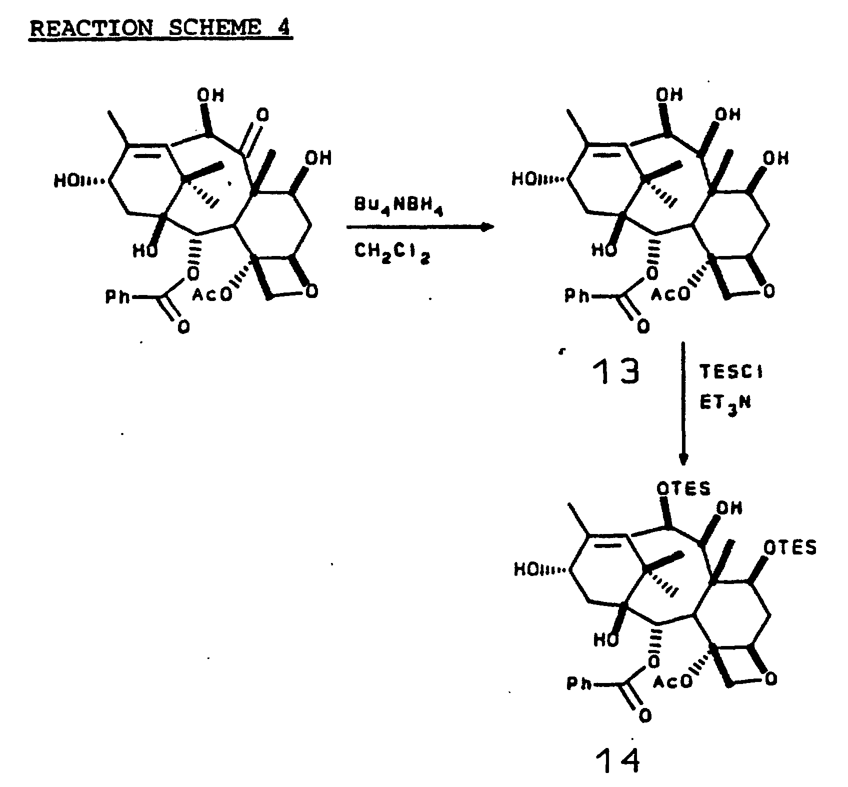

Taxanes having C9 and/or C10 acyloxy substituents other than acetate can be prepared using 10-DAB as a starting material as illustrated in Reaction Scheme 5. Reaction of 10-DAB with triethylsilyl chloride in pyridine yields 7-protected 10-DAB **15**. The C10 hydroxy substituent of 7-protected 10-DAB **15** may then be readily acylated with any standard acylating agent to yield derivative **16** having a new C10 acyloxy substituent. Selective reduction.of the C9 keto substituent of derivative **16** yields 9β-hydroxy derivative **17** to which a C13 side chain may be attached. Alternatively, the C10 and C9 groups can be caused to migrate as set forth in Reaction Scheme **2**, above.

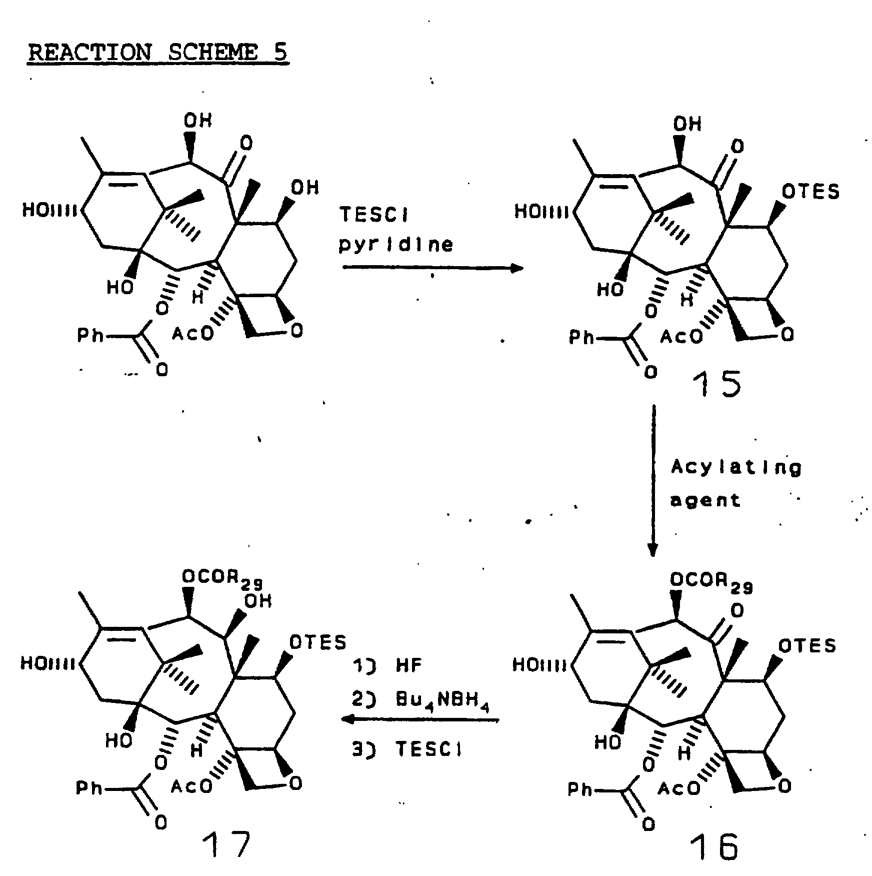

Taxanes having alternative C2 and/or C4 esters can be prepared using baccatin III and 10-DAB as starting materials. The C2 and/or C4 esters of baccatin III and 10-DAB can be selectively reduced to the corresponding alcohol(s) using reducing agents such as LAH or Red-Al, and new esters can thereafter be substituted using standard acylating agents such as anhydrides and acid chlorides in combination with an amine such as pyridine, triethylamine, DMAP, or diisopropyl ethyl amine. Alternatively, the C2 and/or C4 alcohols may be converted to new C2 and/or C4 esters through formation of the corresponding alkoxide by treatment of the alcohol with a suitable base such as LDA followed by an acylating agent such as an acid chloride.

Baccatin III and 10-DAB analogs having different substituents at C2 and/or C4 can be prepared as set forth in Reaction Schemes 6-10. To simplify the description, 10-DAB is used as the starting material. It should be understood, however, that baccatin III derivatives or analogs may be produced using the same series of reactions (except for the protection of the C10 hydroxy group) by simply replacing 10-DAB with baccatin III as the starting material. 9-desoxo derivatives of the baccatin III and 10-DAB analogs having different substituents at C2 and/or C4 can then be prepared by reducing the C9 keto substituent of these analogs and carrying out the other reactions described above.

In Reaction Scheme 6, protected 10-DAB **3** is converted to the triol **18** with lithium aluminum hydride. Triol **18** is then converted to the corresponding C4 ester using Cl₂CO in pyridine followed by a nucleophilic agent (e.g., Grignard reagents or alkyllithium reagents).

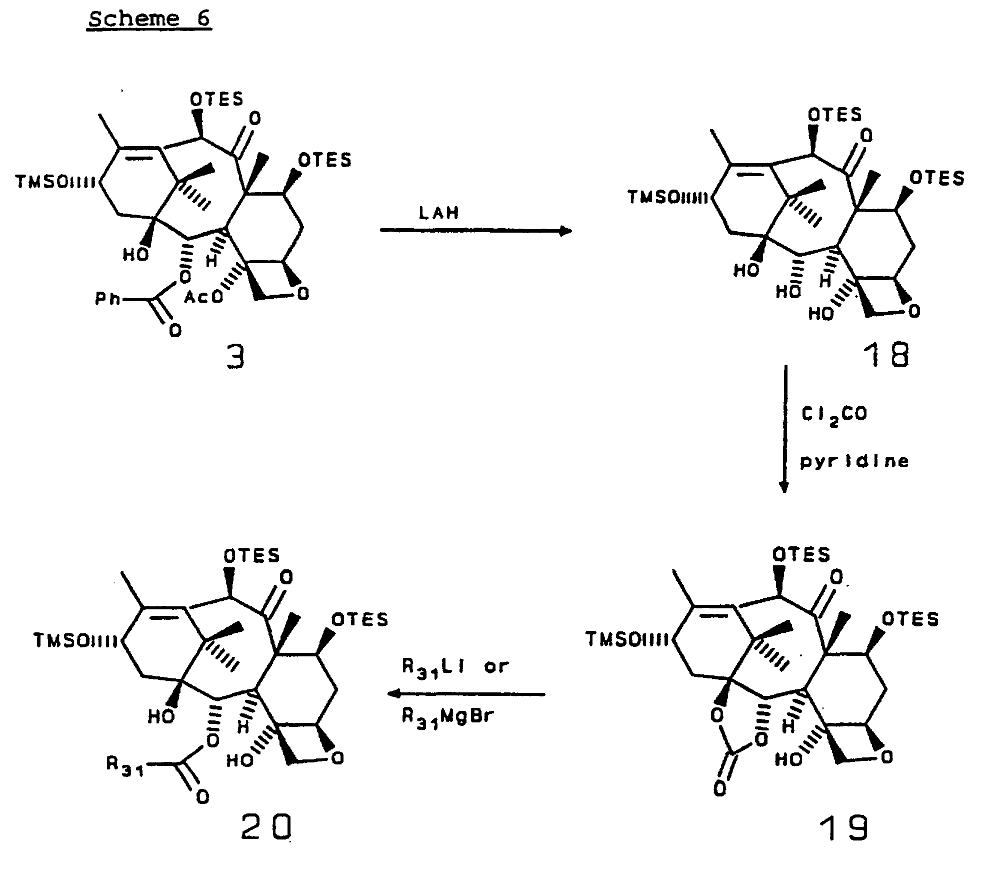

Deprotonation of triol **18** with LDA followed by introduction of an acid chloride selectively gives the C4 ester. For example, when acetyl chloride was used, triol **18** was converted to 1,2 diol **4** as set forth in Reaction Scheme 7.

Triol **18** can also readily be converted to the 1,2 carbonate **19**. Acetylation of carbonate **19** under vigorous standard conditions provides carbonate **21** as described in Reaction Scheme 8; addition of alkyllithiums or Grignard reagents to carbonate **19** provides the C2 ester having a free hydroxyl group at C4 as set forth in Reaction Scheme 6.

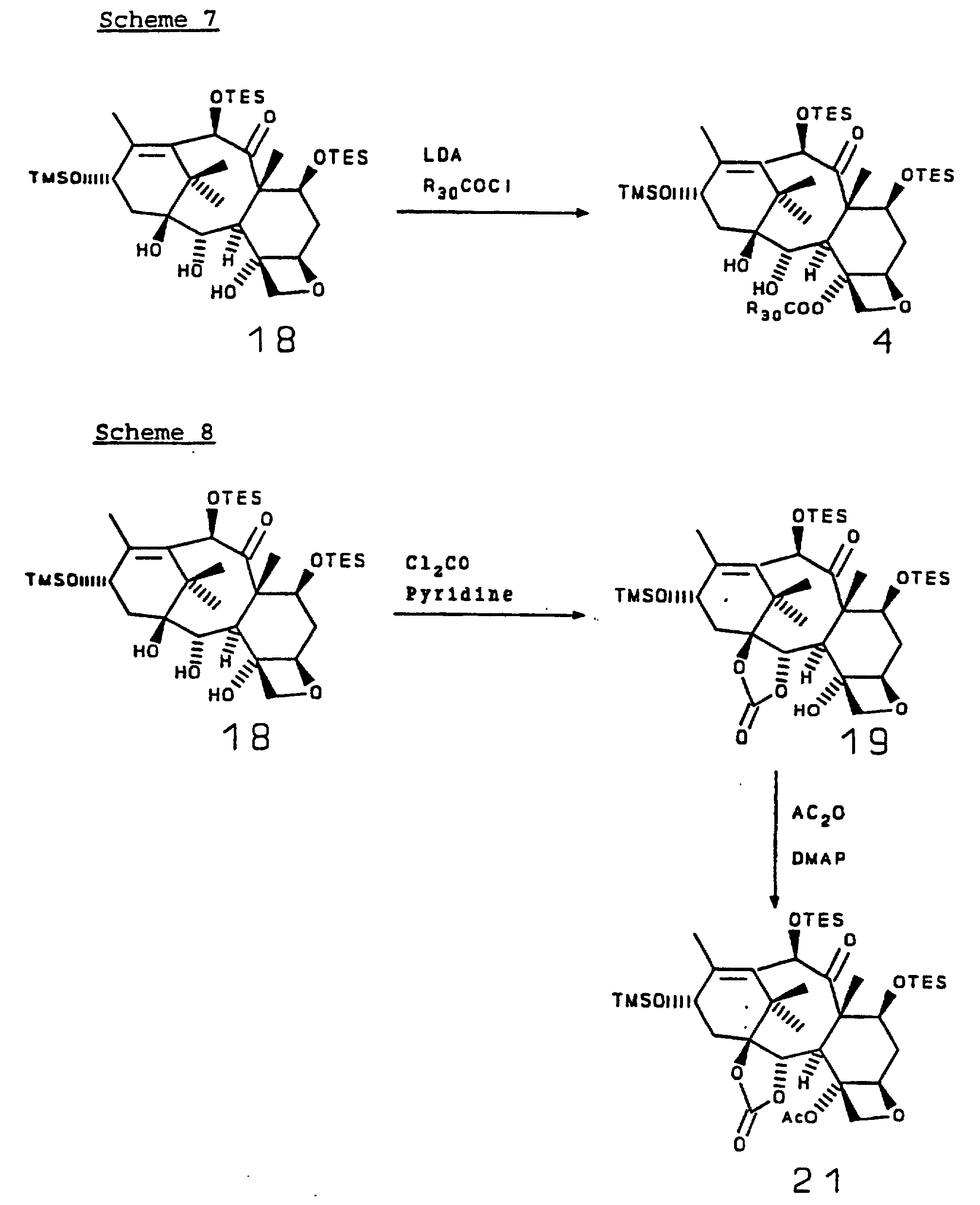

As set forth in Reaction Scheme 9, other C4 substituents can be provided by reacting carbonate **19** with an acid chloride and a tertiary amine to yield carbonate **22** which is then reacted with alkyllithiums or Grignard reagents to provide 10-DAB derivatives having new substituents at C2.

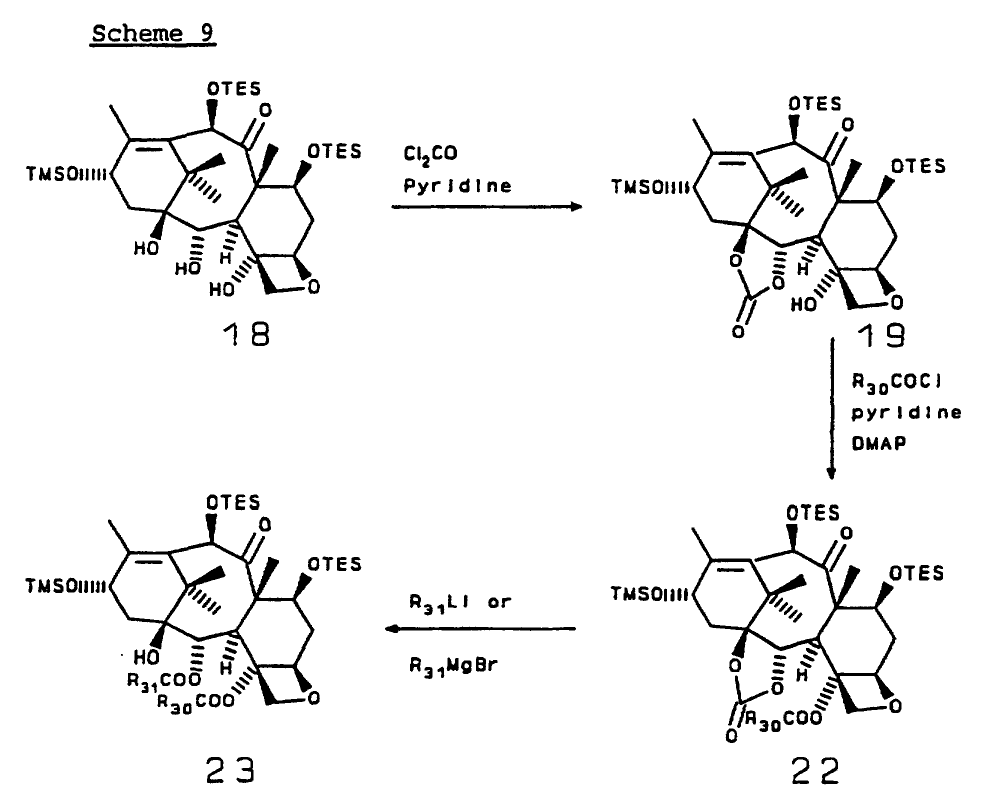

Alternatively, baccatin III may be used as a starting material and reacted as shown in Reaction Scheme 10. After being protected at C7 and C13, baccatin III is reduced with LAH to produce 1,2,4,10 tetraol **24.** Tetraol **24** is converted to carbonate **25** using Cl₂CO and pyridine, and carbonate **25** is acylated at C10 with an acid chloride and pyridine to produce carbonate **26** (as shown) or with acetic anhydride and pyridine (not shown). Acetylation of carbonate **26** under vigorous standard conditions provides carbonate **27** which is then reacted with alkyl lithiums to provide the baccatin III derivatives having new substituents at C2 and C10.

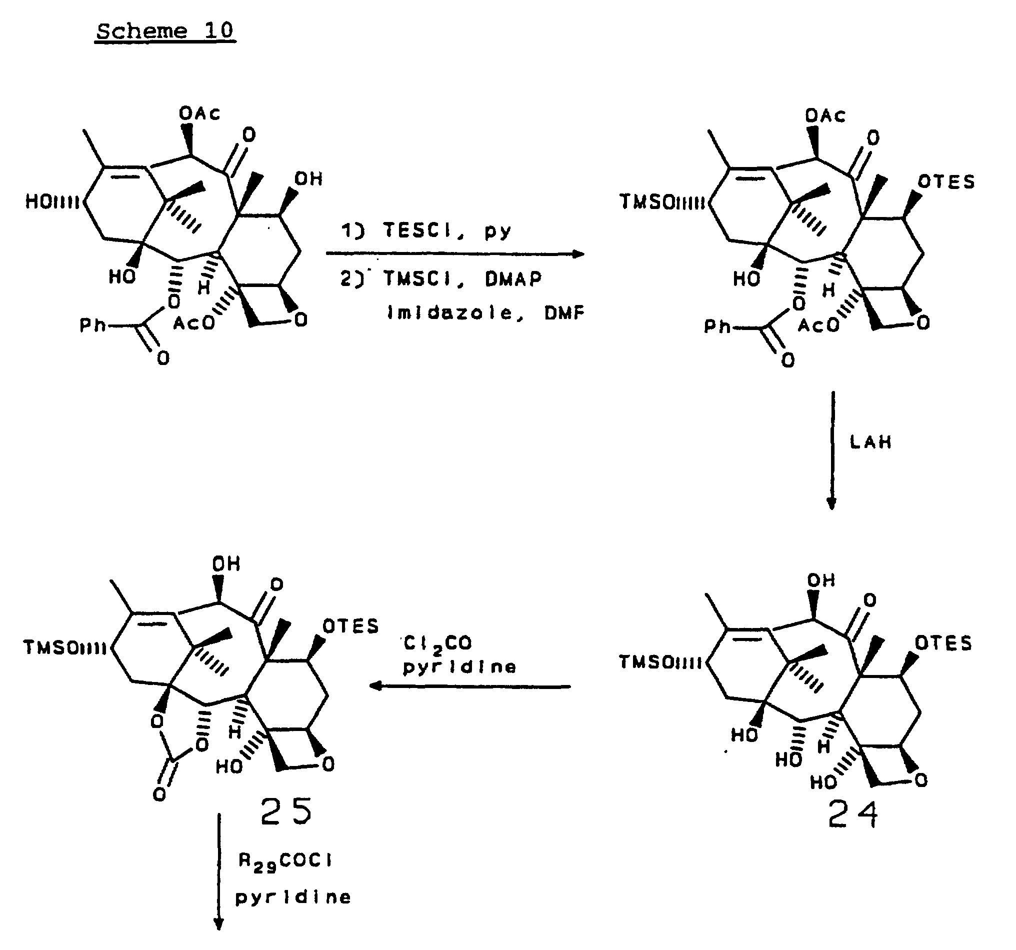

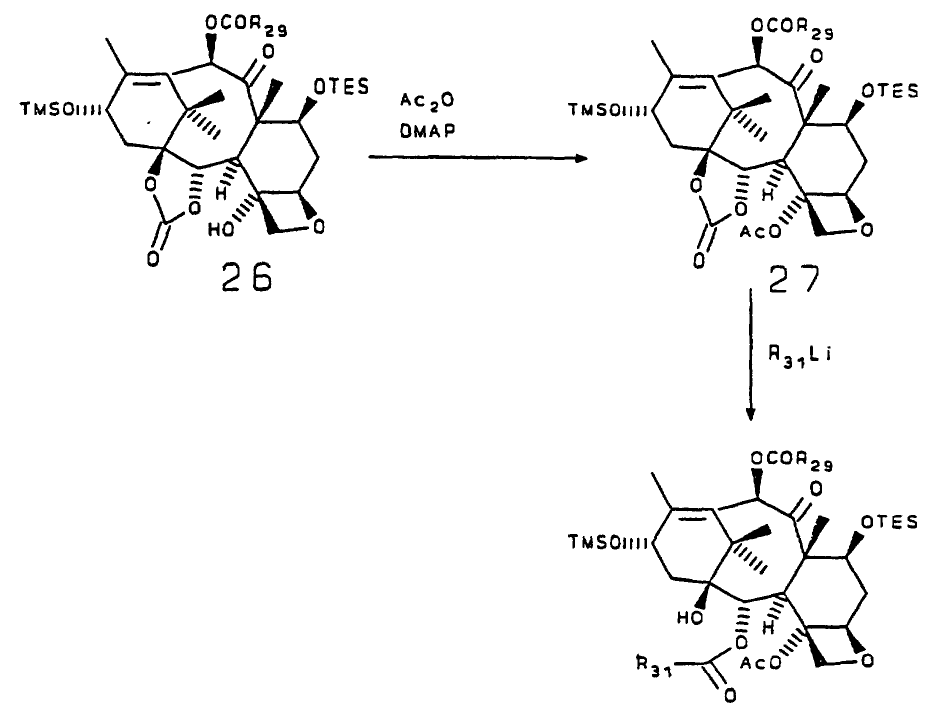

10-desacetoxy derivatives of baccatin III and 10-desoxy derivatives of 10-DAB may be prepared by reacting baccatin III or 10-DAB (or their derivatives) with samarium diiodide. Reaction between the tetracyclic taxane having a C10 leaving group and samarium diiodide may be carried out at 0°C in a solvent such as tetrahydrofuran. Advantageously, the samarium diiodide selectively abstracts the C10 leaving group; C13 side chains and other substituents on the tetracyclic nucleus remain undisturbed. Thereafter, the C9 keto substituent may be reduced to provide the corresponding 9-desoxo-9β-hydroxy-10-desacetoxy or 10-desoxy derivatives as otherwise described herein.

C7 dihydro and other C7 substituted taxanes can be prepared as set forth in Reaction Schemes 11, 12 and 12a.

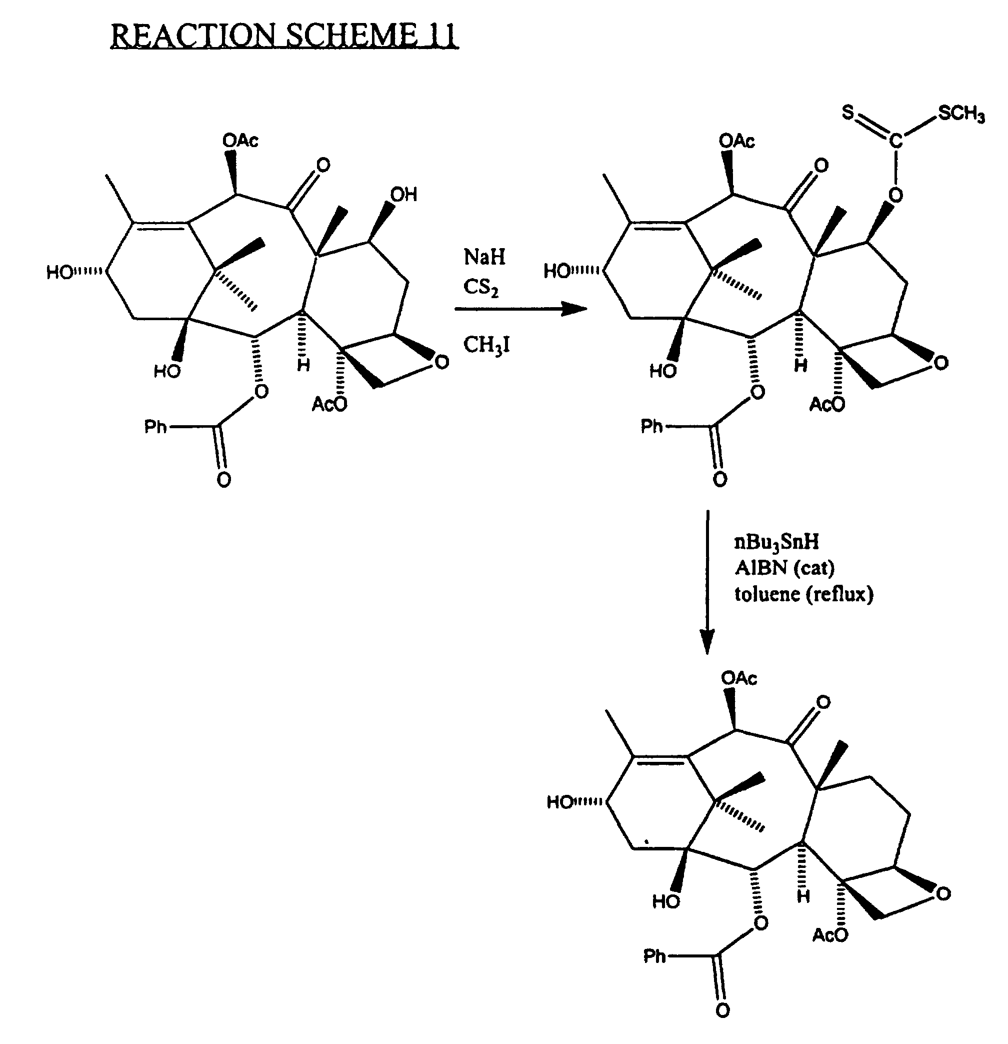

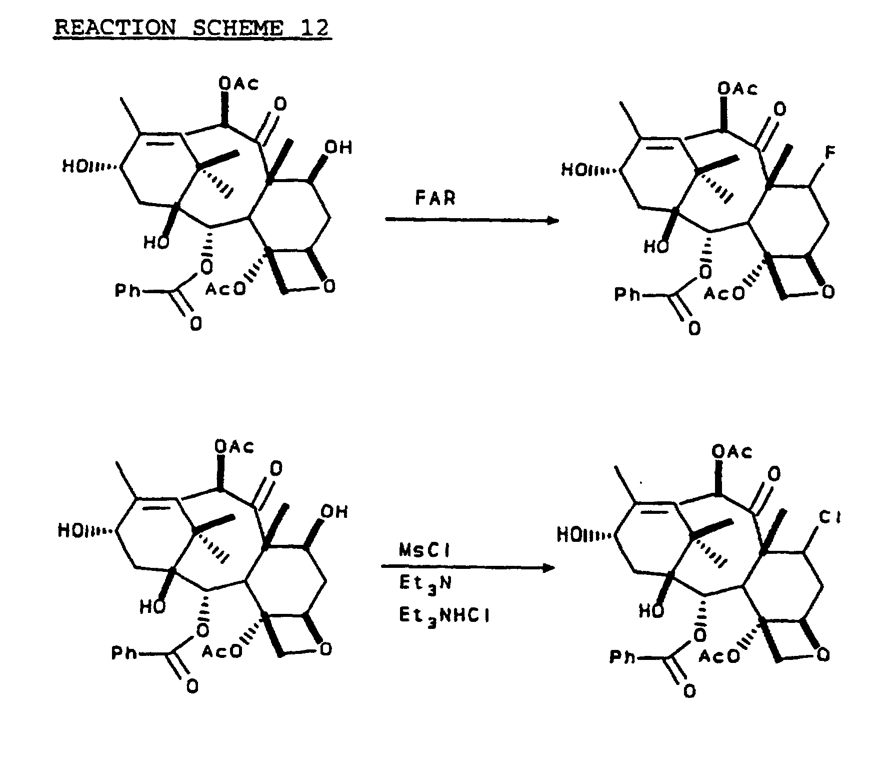

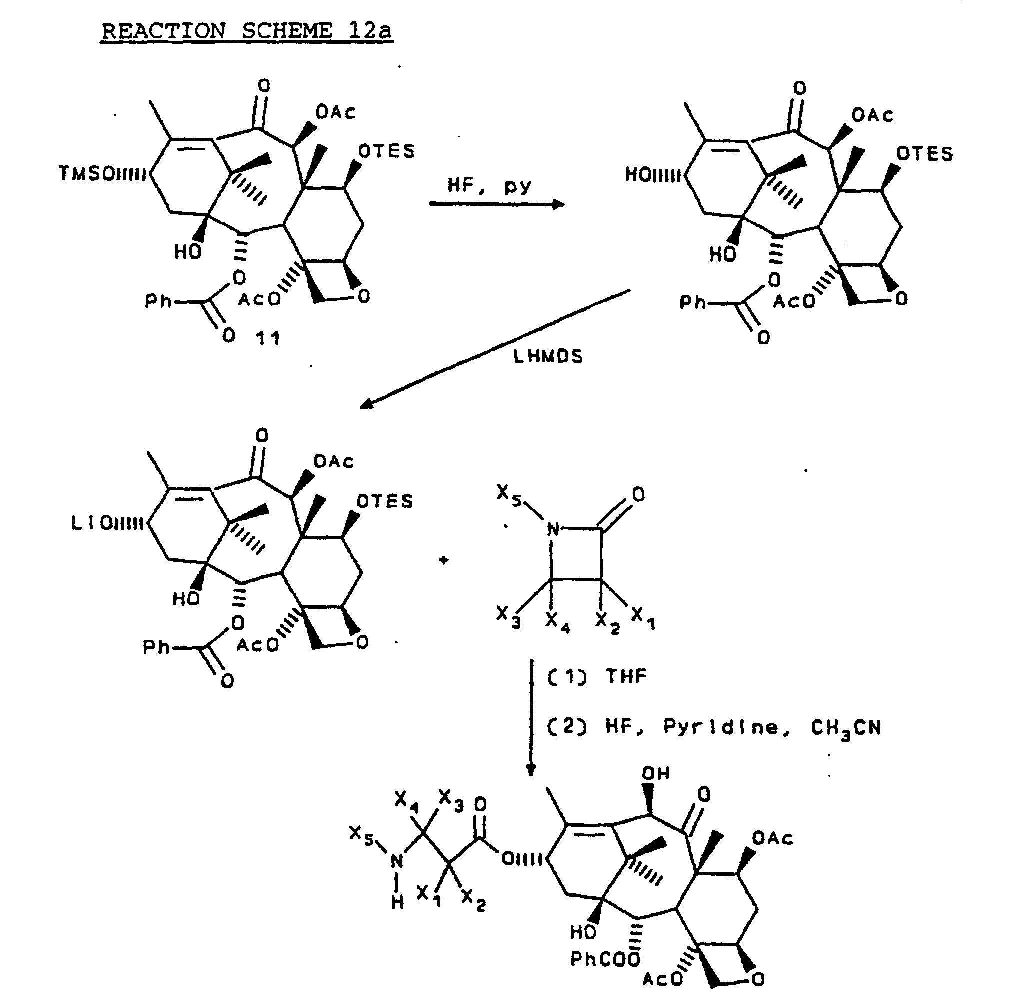

As shown in Reaction Scheme 12, Baccatin III may be converted into 7-fluoro baccatin III by treatment with FAR at room temperature in THF solution. Other baccatin derivatives with a free C7 hydroxyl group behave similarly. Alternatively, 7-chloro baccatin III can be prepared by treatment of baccatin III with methane sulfonyl chloride and triethylamine in methylene chloride solution containing an excess of triethylamine hydrochloride.

Taxanes having C7 acyloxy substituents can be prepared as set forth in Reaction Scheme 12a, 7,13-protected 10-oxo-derivative 11 is converted to its corresponding C13 alkoxide by selectively removing the C13 protecting group and replacing it with a metal such as lithium. The alkoxide is then reacted with a β-lactam or other side chain precursor. Subsequent hydrolysis of the C7 protecting groups causes a migration of the C7 hydroxy substituent to C10, migration of the C10 oxo substituent to C9, and migration of the C9 acyloxy substituent to C7.

A wide variety of tricyclic taxanes are naturally occurring, and through manipulations analogous to those described herein, an appropriate side chain can be attached to the C13 oxygen of these substances. Alternatively, as shown in Reaction Scheme 13, 7-O-triethylsilyl baccatin III can be converted to a tricyclic taxane through the action of trimethyloxonium tetrafluoroborate in methylene chloride solution. The product diol then reacts with lead tetraacetate to provide the corresponding C4 ketone.

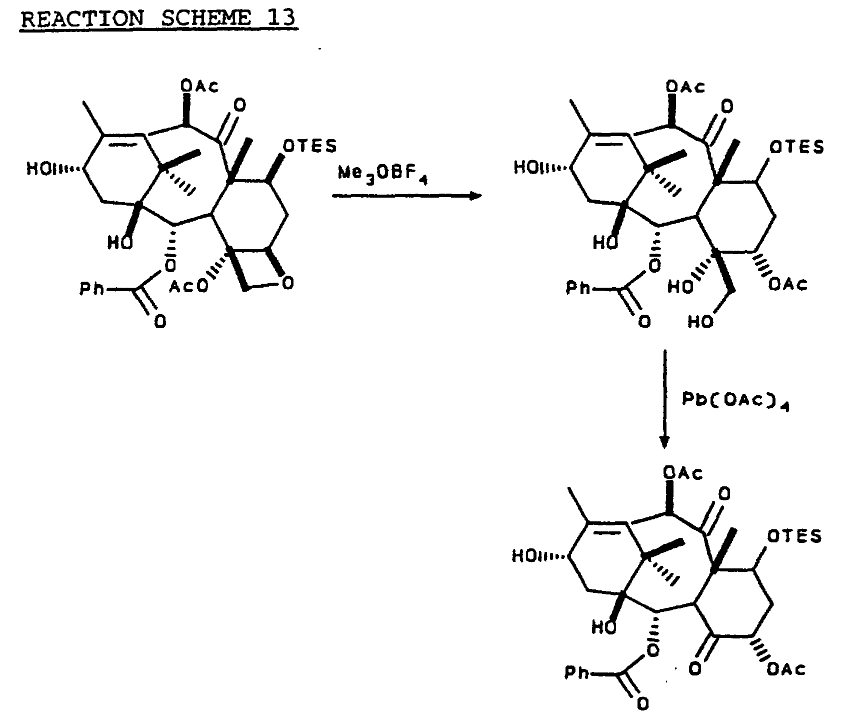

Recently a hydroxylated taxane (14-hydroxy-10-deacetylbaccatin III) has been discovered in an extract of yew needles (C&EN, p 36-37, April 12, 1993). Derivatives of this hydroxylated taxane having the various C2, C4, etc. functional groups described above may also be prepared by using this hydroxylated taxane. In addition, the C14 hydroxy group together with the C1 hydroxy group of 10-DAB can be converted to a 1,2-carbonate as described in C&EN or it may be converted to a variety of esters or other functional groups as otherwise described herein in connection with the C2, C4, C9 and C10 substituents.

The following examples are provided to more fully illustrate the invention.

### EXAMPLE 1

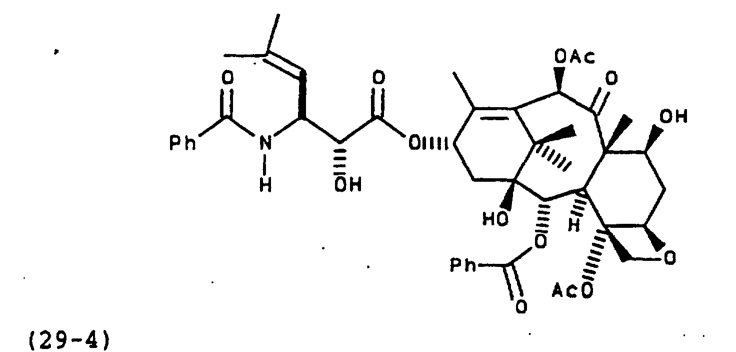

### Preparation of 3'-desphenyl-3'-(2-methyl-1-propenyl) taxol.

To a solution of 7-triethylsilyl baccatin III (120 mg, 0.171 mmol) in 1.2 mL of THF at -45 °C was added dropwise 0.104 mL of a 1.63M solution of nBuLi in hexane. After 0.5 h at -45 °C, a solution of cis-1-benzoyl-3-tri-ethylsilyloxy-4-(2-methyl-1-propenyl)azetidin-2-one (295 mg, 0.885 mmol) in 1.2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 179 mg of a mixture containing (2'R,3'S)-2',7-(bis)triethylsilyl-3'-desphenyl-3'-(2-methyl-1-propenyl) taxol and a small amount of the (2'S,3'R) isomer.

To a solution of 179 mg (0.171 mmol) of the mixture obtained from the previous reaction in 11 mL of acetonitrile and 0.55 mL of pyridine at 0 °C was added 1.7 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 140 mg of material which was purified by flash chromatography to give 109.0 mg (78%) of 3'-desphenyl-3'-(2-methyl-1-propenyl) taxol, which was recrystallized from methanol/water.

m.p.143-144 °C; [α]²⁵_{Na}-61.0° (c 0.0065, CHCl₃).

¹H NMR (CDCl₃, 300 MHz) δ 8.11(d, J=7.1 Hz, 2H, benzoate ortho), 7.69(d, J=8.3 Hz, 2H, benzamide ortho), 7.64-7.36 (m, 6H, aromatic), 6.45(d, J=8.2 Hz, 1H, NH), 6.29(s, 1H, H10), 6.20 (dd, J = 7.7, 7.7 Hz, 1H, H13), 5.68(d, J = 7.1 Hz, 1H, H2β), 5.46(m, 1H, vinyl), 5.27(ddd, J=8.8, 8.8, 3.3 Hz, 1H, H3'), 4.96 (d, J = 7.7 Hz, 1H, H5), 4.40(m, 1H, H7),4.36(m, 1H, H2'), 4.32(d, J= 7.8 Hz, 1H, H20α), 4.22 (d, J=7.8 Hz, 1H, H20β), 3.82(d, J= 7.1 Hz, 1H, H3), 3.63(d, J= 6.6 Hz, 1H, 2'OH), 2.54(m, 1H, H6α), 2.48(d, J=3.9 Hz, 1H, 7OH), 2.42(m, 2H, H14), 2.39(s, 3H, 4Ac),, 2.23 (s, 3H, 10Ac), 2.16(br s, 3H, Me18), 1.89 (m, 1H, H6β), 1.88 (s, 3H, Me19), 1.80(s, 4H, Me thienyl+1OH), 1.24(s, 3H, Me17), 1.14(s, 3H, Me16).

### EXAMPLE 2

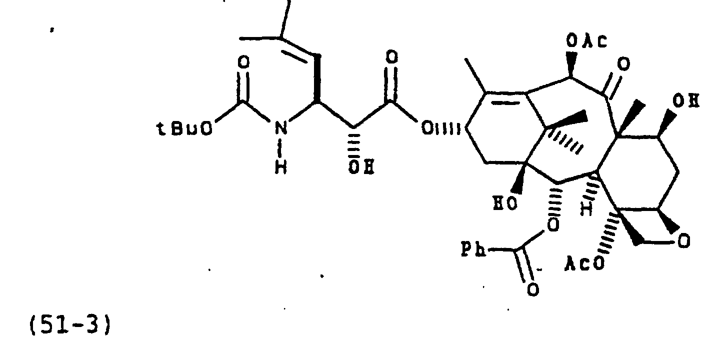

### Preparation of 3'-desphenyl-3'-isobutenyl-N-debenzoyl-N-(t-butoxycarbonyl) taxol.

To a solution of 7-triethylsilyl baccatin III (30.0 mg, 0.043 mmol) in 0.5 mL of THF at -45 °C was added dropwise 0.047 mL of a 1.0 M solution of (TMS)₂NLi in THF. After 0.5 h at -45 °C, a solution of *cis*-1-(t-butoxy-carbonyl)-3-(2-methoxy-2-propoxy)-4-isobutenylazetidin-2-one (44.2 mg, 0.13 mmol) in 0.4 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 40.3 mg of a mixture containing (2'R,3'S)- 2'-(2-methoxy-2-propoxy)-3'-desphenyl-3'-isobutenyl-7-triethylsilyl-N-debenzoyl-N-(t-butoxy-carbonyl) taxol and a small amount of the (2'S,3'R) isomer.

To a solution of 40.3 mg (0.038 mmol) of the mixture obtained from the previous reaction in 2 mL of acetonitrile and 0.1 mL of pyridine at 0 °C was added 0.3 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 34.2 mg of material which was purified by flash chromatography to give 22.4 mg (72%) of 3'-desphenyl-3'-isobutenyl-N-debenzoyl-N-(t-butoxycarbonyl) taxol, which was recrystallized from methanol/water.

m.p.147-149°C; [α]²⁵_{Na} -65.2° (c 0.0023, CHCl₃).

¹H NMR (CDCl₃, 300 MHz) δ 8.11(d, J=7.1 Hz, 2H, benzoate ortho), 7.61(m, 1H, benzoate para), 7.48 (m, 2H, benzoate meta), 6.45(s, 1H, NH), 6.30(d, J= 8.3 Hz, 1H, H10), 6.18 (dd, *J* = 7.7, 7.7 Hz, 1H, H13), 5.68(d, *J* = 7.1 Hz, 1H, H2β), 5.31(m, 1H, vinyl), 5.01(ddd, J=8.8, 8.8, 3.3 Hz, 1H, H3'), 4.95 (d, *J* = 7.7 Hz, 1H, H5), 4.76(m, 1H, H7), 4.43(m, 1H, H2'), 4.32(d, *J* = 7.8 Hz, 1H, H20α), 4.19 (d, J=7.8 Hz, 1H, H20β), 3.81(d, J= 7.1 Hz, 1H, H3), 3.74(d, J= 6.6 Hz, 1H, 2'OH), 2.54(m, 1H, H6α), 2.48(d, J=3.9 Hz, 1H, 7OH), 2.44(m, 2H, H14), 2.39(s, 3H, 4Ac), 2.26(s, 3H, Me vinyl), 2.25(s, 3H, Me vinyl), 2.23 (s, 3H, 10Ac), 1.98(br s, 3H, Me18), 1.86 (m, 1H, H6β), 1.76 (s, 3H, Me19), 1.43(s, 9H, 3Me t-butoxy) 1.25(s, 3H, Me17), 1.14(s, 3H, Me16).

### EXAMPLE 3

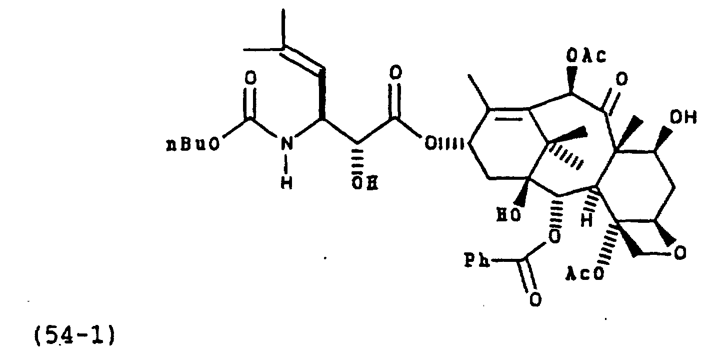

### Preparation of N-debenzoyl-N-(n-butoxycarbonyl)-3'-desphenyl-3'-isobutenyl taxol.

To a solution of 7-triethylsilyl baccatin III (70.0 mg, 0.086 mmol) in 0.7 mL of THF at -45°C was added dropwise 0.10 mL of a 1.0 M solution of LiN(SiMe₃)₂ in hexane. After 0.5 h at -45°C, a solution of *cis*-1-(n-butoxycarbonyl)-3-(2-methoxy-2-propoxy)-4-isobutenyl-azetidin-2-one (94.2mg, 0.214 mmol) in 0.5 mL of THF was added dropwise to the mixture. The solution was warmed to 0°C and kept at that temperature for 1 h before 1.0 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 82.8 mg of a mixture containing (2'R,3'S)-2'-(2-methoxy-2-propoxy)-7-triethylsilyl-N-debenzoyl-N-(n-butoxycarbonyl)-3'-desphenyl-3'-iso-butenyl taxol and a small amount of the (2'S,3'R) isomer.

To a solution of 82.8 mg (0.083 mmol) of the mixture obtained from the previous reaction in 6.0 mL of acetonitrile and 0.3 mL of pyridine at 0°C was added 0.7 mL of 48% aqueous HF. The mixture was stirred at 0°C for 3 h, then at 25°C for 13 h, and partitioned between saturated aqueous sodium bicarbonate and ethyl acetate. Evaporation of the ethyl acetate solution gave 67.7 mg of material which was purified by flash chromatography to give 53.2 mg (77%) of N-debenzoyl-N-(n-butoxycarbonyl)-3'-desphenyl-3'-isobutenyl taxol, which was recrystallized from methanol/water.

m.p.132-134°C; [α]²⁵_{Na} -64.0° (c 0.0023, CHCl₃).

¹H NMR (CDCl₃, 300 MHz) δ 8.11(d, J=7.2 Hz, 2H, benzoate ortho), 7.61(m, 1H, benzoate para), 7.48(m, 2H, benzoate meta), 6.30(s, 1H, H10), 6.21(dd, J = 7.5, 7.5 Hz, 1H, H13), 5.67(d, J = 7.2 Hz, 1H, H2β), 5.33(m, 1H, olefine of isobutenyl), 4.97(d, J= 7.8, 1H, H5), 4.91(d, J=8.2 Hz, 1H, NH), 4.78(ddd, J=8.7, 8.7, 2.7 Hz, 1H, H3'), 4.43(m 1H, H2'), 4.31(d, J=7.8 Hz, 1H, H20α), 4.25(m, 1H, H7), 4.16(d, J=7.8 Hz, 1H, H20β), 3.96(q, J=6.6 Hz, 2H, n-butyloxy), 3.81(d, J= 7.2 Hz, 1H, H3), 3.34(d, J= 6.6 Hz, 1H, 2'OH), 2.54(m, 1H, H6α), 2.50(d, J=3.9 Hz, 1H, 7OH), 2.36(s, 3H, 4Ac), 2.33(m, 2H, H14), 2.26(s, 3H, 10Ac), 2.24(br s, 3H, Me18), 1.89(s, 3H, Me19), 1.87(m, 1H, H6β), 1.77(s, 3H, Me isobutenyl), 1.75(s, 1H, 1OH), 1.68(s, 3H, Me isobutenyl), 1.56(m, 2H, n-butyloxy), 1.32(m, 2H, n-butyloxy), 1.26(s, 3H, Me17), 1.15(s, 3H, Me16), 0.85(t, J=6.6 Hz, 3H, Me of n-butyloxy).

### EXAMPLE 4

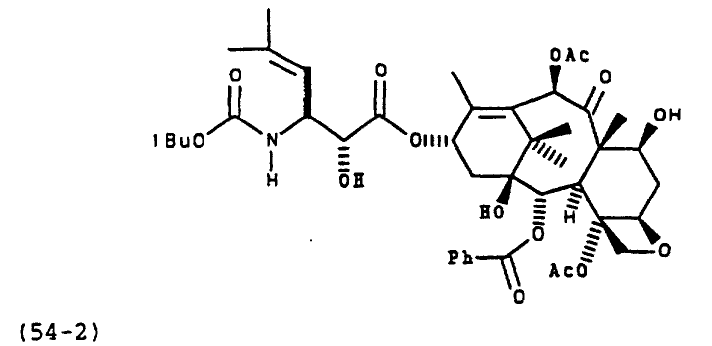

### Preparation of 3'-desphenyl-3'-isobutenyl-N-debenzoyl-N-(isobutyloxycarbonyl) taxol.

To a solution of 7-triethylsilyl baccatin III (40.0 mg, 0.042 mmol) in 0.5 mL of THF at -45°C was added dropwise 0.05 mL of a 1.0 M solution of LiN(SiMe₃)₂ in hexane. After 0.5 h at -45 °C, a solution of *cis*-1-(isobutyloxycarbonyl)-3-(2-methoxy-2-propoxy)-4-iso-butenylazetidin-2-one (43.0 mg, 0.13 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 31.2 mg of a mixture containing (2'R,3'S)-2'-(2-methoxy-2-propoxy)-7-triethylsilyl-3-desphenyl-3'-isobutenyl-N-debenzoyl-N-(isobutyloxycarbonyl) taxol and a small amount of the (2'S,3'R) isomer.

To a solution of 31.2 mg (0.030 mmol) of the mixture obtained from the previous reaction in 2.0 mL of acetonitrile and 0.12 mL of pyridine at 0 °C was added 0.25 mL of 48% aqueous HF. The mixture was stirred at 0°C for 3 h, then at 25 °C for 13 h, and partitioned between saturated aqueous sodium bicarbonate and ethyl acetate. Evaporation of the ethyl acetate solution gave 27.7 mg of material which was purified by flash chromatography to give 20.7 mg (83%) of 3'-desphenyl-3'-isobutenyl-N-debenzoyl-N-(isobutyloxycarbonyl) taxol, which was recrystallized from methanol/water.

m.p.147-148°C; [α]²⁵_{Na} -58.2° (c 0.0016, CHCl₃).

¹H NMR (CDCl₃, 300 MHz) δ 8.11(d, J=7.2 Hz, 2H, benzoate ortho), 7.61(m, 1H, benzoate para), 7.50(m, 2H, benzoate meta), 6.30(s, 1H, H10), 6.22(dd, J = 7.5, 7.5 Hz, 1H, H13), 5.65(d, J = 7.2 Hz, 1H, H2β), 5.31(m, 1H, olefine of isobutenyl), 4.95(d, J= 7.8, 1H, H5), 4.91(d, J=8.2 Hz, 1H, NH), 4.76(ddd, J=8.7, 8.7, 2.7 Hz, 1H, H3'), 4.41(m, 1H, H2'), 4.33(d, J=7.8 Hz, 1H, H20α), 4.25(m, 1H, H7), 4.16(d, J=7.8 Hz, 1H, H20β), 3.81(d, J= 7.2 Hz, 1H, H3), 3.71(dd, J=10.2, 6.6 Hz, 1H, isobutyl), 3.60(dd, J=10.2, 6.6 Hz, 1H, isobutyl), 3.31(d, J= 6.6 Hz, 1H, 2'OH), 2.55(m, 1H, H6α), 2.50(d, J=3.9 Hz, 1H, 7OH), 2.37(s, 3H, 4Ac), 2.31(m, 2H, H14), 2.26(s, 3H, 10Ac), 2.23(br s, 3H, Me18), 1.89(s, 3H, Me19), 1.87(m, 1H, H6β), 1.77(s, 3H, Me isobutenyl), 1.75(s, 1H, 1OH). 1.66(s, 3H, Me isobutenyl), 1.25(s, 3H, Me17), 1.15(s, 3H, Me16), 0.76(d, J=7.2 Hz, 3H, Me of isobutyl), 0.70(d, J=6.6 Hz, 3H, Me of isobutyl).

### EXAMPLE 5

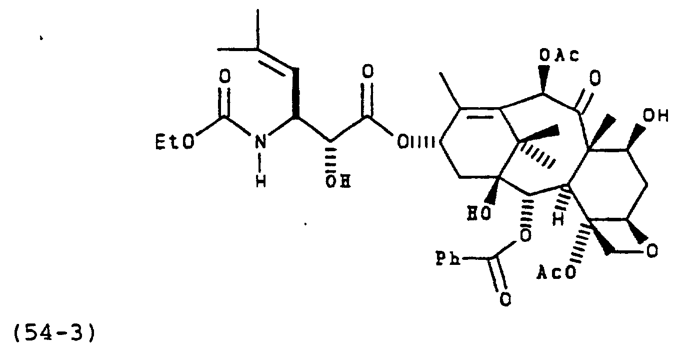

### Preparation of 3'-desphenyl-3'-isobutenyl-N-debenzoyl-N-(ethoxycarbonyl) taxol.

To a solution of 7-triethylsilyl baccatin III (100.0 mg, 0.142 mmol) in 1.0 mL of THF at -45°C was added dropwise 0.16 mL of a 1.0 M solution of LiN(SiMe₃)₂ in hexane. After 0.5 h at -45 °C, a solution of *cis*-1-(ethoxycarbonyl)-3-(2'-2"-(2-methoxy-2-propoxy)-4-isobute nyl-azetidin-2-one (155 mg, 0.43 mmol) in 1.0 mL of THF was added dropwise to the mixture. The solution was warmed to 0 °C and kept at that temperature for 1 h before 1.0 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 112.2 mg of a mixture containing (2'R,3'S)-2'-(2-methoxy-2-propoxy)-3'-desphenyl-3'-isobutenyl-7-triethylsilyl-N-debenzoyl-N-(ethoxycarbonyl) taxol and a small amount of the (2'S,3'R) isomer.

To a solution of 112.2 mg (0.109 mmol) of the mixture obtained from the previous reaction in 7.0 mL of acetonitrile and 0.4 mL of pyridine at 0 °C was added 0.9 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 98.7 mg of material which was purified by flash chromatography to give 81.4 mg (93%) of 3'-desphenyl-3'-isobutenyl-N-debenzoyl-N-(ethoxycarbonyl) taxol, which was recrystallized from methanol/water.

m.p.137-140°C; [α]²⁵_{Na} -56.2.0° (c 0.0023, CHCl₃).

¹H NMR (CDCl₃, 300 MHz) δ 8.11(d, J=7.2 Hz, 2H, benzoate ortho), 7.61(m, 1H, benzoate para), 7.50(m, 2H, benzoate meta), 6.30(s, 1H, H10), 6.19(dd, J = 7.5, 7.5 Hz, 1H, H13), 5.65(d, J = 7.2 Hz, 1H, H2β), 5.31(m, 1H, olefine of isobutenyl), 4.98(d, J= 7.8, 1H, H5), 4.90(d, J=8.2 Hz, 1H, NH), 4.75(ddd, J=8.7, 8.7, 2.7 Hz, 1H, H3'), 4.45(m, 1H, H2'), 4.31(d, J=7.8 Hz, 1H, H20α), 4.25(m, 1H, H7), 4.16(d, J=7.8 Hz, 1H, H20β), 3.93(q, J=7.2 Hz, 2H, ethyl), 3.81(d, J= 7.2 Hz, 1H, H3), 3.34(d, J= 6.6 Hz, 1H, 2'OH), 2.54(m, 1H, H6α), 2.50(d, J=3.9 Hz, 1H, 7OH), 2.36(s, 3H, 4Ac), 2.33(m, 2H, H14), 2.26(s, 3H, 10Ac), 2.24(br s, 3H, Me18), 1.89(s, 3H, Me19), 1.87(m, 1H, H6β), 1.78(s, 3H, Me isobutenyl), 1.73(s, 1H, 1OH), 1.68(s, 3H, Me isobutenyl), 1.26(s, 3H, Me17), 1.15(s, 3H, Me16), 1.08(t, J=7.2 Hz, 3H, Me of ethyl).

### EXAMPLE 6

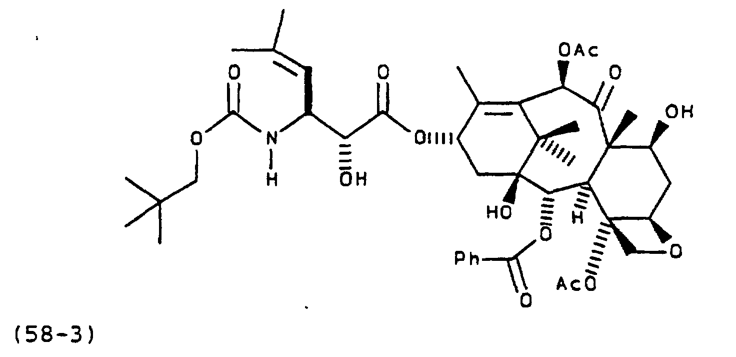

### Preparation of 3'-desphenyl-3'-isobutenyl-N-debenzoyl-N-(neopentyloxycarbonyl) taxol.

To a solution of 7-triethylsilyl baccatin III (50.0 mg, 0.071 mmol) in 0.7 mL of THF at -45 °C was added dropwise 0.08 mL of a 1.0 M solution of LiN(SiMe₃)₂ in hexane. After 0.5 h at -45 °C, a solution of *cis*-1-(neopentyloxycarbonyl)-3-(2-methoxy-2-propoxy)-4-iso-buthenylazetidin-2-one (68.9 mg, 0.21 mmol) in 1.0 mL of THF was added dropwise to the mixture. The solution was warmed to 0 °C and kept at that temperature for 1 h before 1.0 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 65.1 mg of a mixture containing (2'R,3'S)-2'-(2-methoxy-2-propoxy)-3'-desphenyl-3'-isobutenyl-7-triethylsilyl-N-debenzoyl-N-(neopentyloxycarbonyl) taxol and a small amount of the (2'S,3'R) isomer.

To a solution of 65.1 mg (0.057 mmol) of the mixture obtained from the previous reaction in 6.0 mL of acetonitrile and 0.3 mL of pyridine at 0 °C was added 0.7 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 58.2 mg of material which was purified by flash chromatography to give 31.2 mg (65%) of 3'-desphenyl-3'-(isobutenyl)-N-debenzoyl-N-(neopentyloxycarbonyl) taxol, which was recrystallized from methanol/water.

m.p.147-149°C; [α]²⁵_{Na} -58.5° (c 0.0019, CHCl₃).

¹H NMR (CDCl₃, 300 MHz) δ 8.15(d, J=7.2 Hz, 2H, benzoate ortho), 7.61(m, 1H, benzoate para), 7.50(m, 2H, benzoate meta), 6.30(s, 1H, H10), 6.22(dd, J = 7.5, 7.5 Hz, 1H, H13), 5.68(d, J = 7.2 Hz, 1H, H2β), 5.32(m, 1H, olefine of isobutenyl), 4.98(d, J= 7.8, 1H, H5), 4.89(d, J=8.2 Hz, 1H, NH), 4.76(ddd, J=8.7, 8.7, 2.7 Hz, 1H, H3'), 4.43(m, 1H, H2'), 4.29(d, J=7.8 Hz, 1H, H20α), 4.25(m, 1H, H7), 4.16(d, J=7.8 Hz, 1H, H20β), 3.76(s, 2H, neopentyloxy), 3.81(d, J= 7.2 Hz, 1H, H3), 3.34(d, J= 6.6 Hz, 1H, 2'OH), 2.55(m, 1H, H6α), 2.50(d, J=3.9 Hz, 1H, 7OH), 2.33(s, 3H, 4Ac), 2.30(m, 2H, H14), 2.26(s, 3H, 10Ac), 2.24(br s, 3H, Me18), 1.89(s, 3H, Me19), 1.87(m, 1H, H6β), 1.77(s, 3H, Me isobutenyl), 1.75(s, 1H, 10H), 1.68(s, 3H, Me isobutenyl), 1.26(s, 3H, Me17), 1.20(s, 9H, Me of neopentyloxy) 1.15(s, 3H, Me16).

### EXAMPLE 7

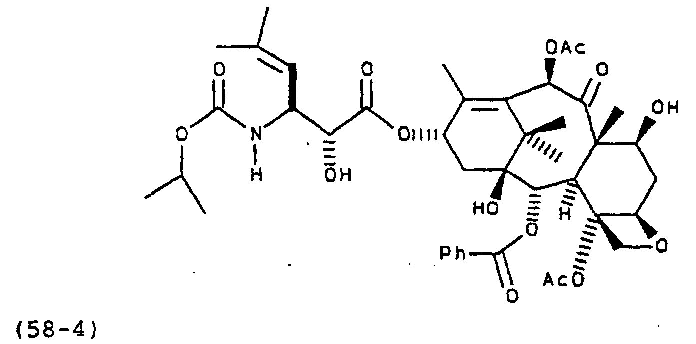

### Preparation of 3'-desphenyl-3'-isobutenyl-N-debenzoyl-N-(isopropyloxycarbonyl) taxol.

To a solution of 7-triethylsilyl baccatin III (50.0 mg, 0.071 mmol) in 0.7 mL of THF at -45 °C was added dropwise 0.08 mL of a 1.0 M solution of LiN(SiMe₃)₂ in hexane. After 0.5 h at -45 °C, a solution of *cis*-1-(isopropyloxycarbonyl) -3-(2-methoxy-2-propoxy)-4-isobutenylazetidin-2-one (56.3 mg, 0.22 mmol) in 1.0 mL of THF was added dropwise to the mixture. The solution was warmed to 0°C and kept at that temperature for 1 h before 1.0 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 63.4 mg of a mixture containing (2'R,3'S)-2'-(2-methoxy-2-propoxy)-3'-desphenyl-3'-isobutenyl-7-triethylsilyl-N-debenzoyl-N-(isopropyloxycarbonyl) taxol and a small amount of the (2'S,3'R) isomer.

To a solution of 63.4 mg (0.057 mmol) of the mixture obtained from the previous reaction in 5.5 mL of acetonitrile and 0.3 mL of pyridine at 0 °C was added 0.66 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 49.2 mg of material which was purified by flash chromatography to give 38.2 mg (82%) of 3'-desphenyl-3'-isobutenyl-N-debenzoyl-N-(isopropyloxycarbonyl) taxol, which was recrystallized from methanol/water.

m.p.145-147°C; [α]²⁵_{Na} -58.3° (c 0.0019, CHCl₃).

¹H NMR (CDCl₃, 300 MHz) δ 8.12(d, J=7.2 Hz, 2H, benzoate ortho), 7.61(m, 1H, benzoate para), 7.50(m, 2H, benzoate meta), 6.30(s, 1H, H10), 6.20(dd, J = 7.5, 7.5 Hz, 1H, H13), 5.65(d, J = 7.2 Hz, 1H, H2β), 5.31(m, 1H, olefine of isobutenyl), 4.96(d, J= 7.8, 1H, H5), 4.90(d, J=8.2 Hz, 1H, NH), 4.77(ddd, J=8.7, 8.7, 2.7 Hz, 1H, H3'), 4.69(m, 1H, isopropyloxy), 4.43(m, 1H, H2'), 4.31(d, J=7.8 Hz, 1H, H20α), 4.24(m, 1H, H7), 4.15(d, J=7.8 Hz, 1H, H20β), 3.81(d, J= 7.2 Hz, 1H, H3), 3.33(d, J= 6.6 Hz, 1H, 2'OH), 2.54(m, 1H, H6α), 2.50(d, J=3.9 Hz, 1H, 7OH), 2.34(s, 3H, 4Ac), 2.30(m, 2H, H14), 2.24(s, 3H, 10Ac), 2.21(br s, 3H, Me18), 1.88(s, 3H, Me19), 1.87(m, 1H, H6β), 1.77(s, 3H, Me isobutenyl), 1.75(s, 1H, 1OH), 1.66(s, 3H, Me isobutenyl), 1.25(s, 3H, Me17), 1.16(s, 3H, Me16), 1.14(d, J=6.6Hz, 3H, Me of isopropyloxy), 1.12(d, J=6.6 Hz, 3H, Me of isopropyloxy).

### EXAMPLE 8

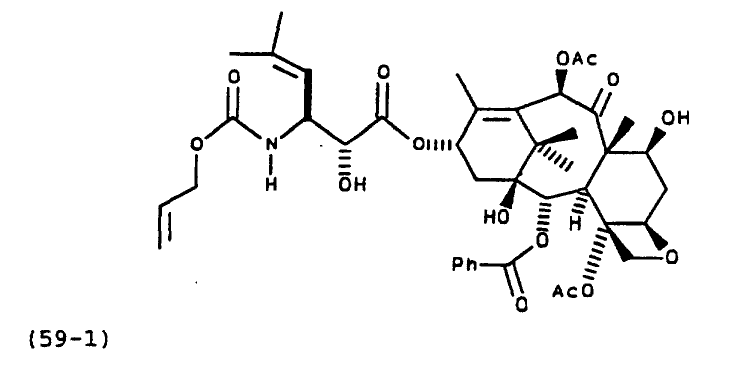

### Preparation of 3'-desphenyl-3'-isobutenyl-N-debenzoyl-N-(allyloxycarbonyl) taxol.

To a solution of 7-triethylsilyl baccatin III (50.0 mg, 0.071 mmol) in 0.7 mL of THF at -45 °C was added dropwise 0.08 mL of a 1.0 M solution of LiN(SiMe₃)₂ in hexane. After 0.5 h at -45 °C, a solution of *cis*-1-(allyloxycarbonyl)-3-(2-methoxy-2-propoxy)-4-isobutenylazetidin-2-one (65.4 mg, 0.22 mmol) in 1.0 mL of THF was added dropwise to the mixture. The solution was warmed to 0°C and kept at that temperature for 1 h before 1.0 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 64.4 mg of a mixture containing (2'R,3'S)-2'-(2-methoxy-2-propoxy)-3'-desphenyl-3'-isobutenyl-7-triethylsilyl-N-debenzoyl-N-(allyloxycarbonyl) taxol and a small amount of the (2'S,3'R) isomer.

To a solution of 64.4 mg (0.058 mmol) of the mixture obtained from the previous reaction in 6.0 mL of acetonitrile and 0.28 mL of pyridine at 0 °C was added 0.7 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 53.2 mg of material which was purified by flash chromatography to give 33.3 mg (71%) of 3'-desphenyl-3'-isobutenyl-N-debenzoyl-N-(allyloxycarbonyl) taxol, which was recrystallized from methanol/water.

m.p.137-139°C; [α]²⁵_{Na} -59.1° (c 0.0022, CHCl₃).

¹H NMR (CDCl₃, 300 MHz) δ 8.11(d, J=7.2 Hz, 2H, benzoate ortho), 7.60(m, 1H, benzoate para), 7.50(m, 2H, benzoate meta), 6.29(s, 1H, H10), 6.21(dd, J = 7.5, 7.5 Hz, 1H, H13), 5.78(m, 1H, allyl), 5.67(d, J = 7.2 Hz, 1H, H2β), 5.33(m, 1H, olefine of isobutenyl), 5.14(m, 2H, allyl), 4.97(d, J= 7.8, 1H, H5), 4.91(d, J=8.2 Hz, 1H, NH), 4.78(ddd, J=8.7, 8.7, 2.7 Hz, 1H, H3'), 4.43(m, 1H, H2'), 4.31(d, J=7.8 Hz, 1H, H20α), 4.25(m, 1H, H7), 4.18(d, J=7.8 Hz, 1H, H20β), 4.08(d, J=6.6 Hz, 2H, allyl) 3.79(d, J= 7.2 Hz, 1H, H3), 3.34(d, J= 6.6 Hz, 1H, 2'OH), 2.55(m, 1H, H6α), 2.50(d, J=3.9 Hz, 1H, 7OH), 2.36(s, 3H, 4Ac), 2.33(m, 2H, H14), 2.26(s, 3H, 10Ac), 2.24(br s, 3H, Me18), 1.88(s, 3H, Me19), 1.85(m, 1H, H6β), 1.72(s, 3H, Me isobutenyl), 1.69(s, 1H, 10H), 1.61(s, 3H, Me isobutenyl), 1.25(s, 3H, Me17), 1.15(s, 3H, Me16).

### EXAMPLE 9

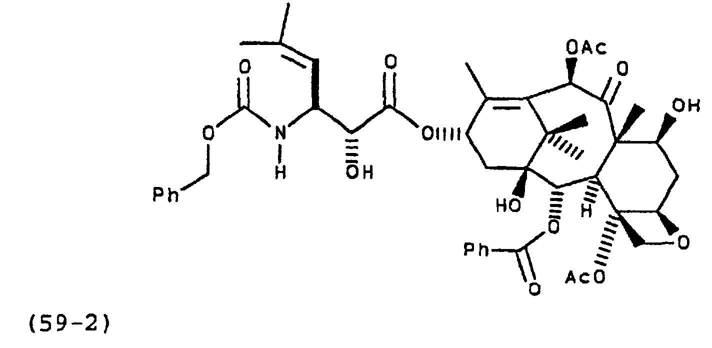

### Preparation of 3'-desphenyl-3'-isobutenyl-N-debenzoyl-N-(benzyloxycarbonyl) taxol.

To a solution of 7-triethylsilyl baccatin III (50.0 mg, 0.071 mmol) in 0.7 mL of THF at -4°C was added dropwise 0.08 mL of a 1.0 M solution of LiN(SiMe₃)₂ in hexane. After 0.5 h at -4°C, a solution of *cis*-1-(benzyloxycarbonyl)-3-(2-methoxy-2-propoxy)-4-iso-butenylazetidin-2-one (63 mg, 0.21 mmol) in 0.5 mL of THF was added dropwise to the mixture. The solution was warmed to 0°C and kept at that temperature for 1 h before 1.0 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 60.4 mg of a mixture containing (2'R,3'S)-2'-(2-methoxy-2-propoxy)-3'-desphenyl-3'-isobutenyl-7-triethylsilyl-N-debenzoyl-N-(benzyloxycarbonyl) taxol and a small amount of the (2'S,3'R) isomer.

To a solution of 60.4 mg (0.053 mmol) of the mixture obtained from the previous reaction in 5.0 mL of acetonitrile and 0.3 mL of pyridine at 0 °C was added 0.65 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 48.2 mg of material which was purified by flash chromatography to give 34.1 mg (74%) of 3'-desphenyl-3'-isobutenyl-N-debenzoyl-N-(benzyloxycarbonyl) taxol, which was recrystallized from methanol/water.

m.p.148-149°C; [α]²⁵_{Na} -53.2.0° (c 0.0026, CHCl₃).

¹H NMR (CDCl₃, 300 MHz) δ 8.15(d, J=7.2 Hz, 2H, benzoate ortho), 7.61(m, 1H, benzoate para), 7.48(m, 2H, benzoate meta), 7.22-7.20 (m, 3H, benzyl), 7.10-7.05(m, 2H, benzyl), 6.29(s, 1H, H10), 6.21(dd, J = 7.5, 7.5 Hz, 1H, H13), 5.63(d, J = 7.2 Hz, 1H, H2b), 5.33(m, 1H, olefine of isobutenyl), 5.06(d, J=12.3 Hz, 1H, benzyl), 4.97(d, J= 7.8, 1H, H5), 4.91(d, J=8.2 Hz, 1H, NH), 4.85(d, J=12.3 Hz, 1H, benzyl), 4.76(ddd, J=8.7, 8.7, 2.7 Hz, 1H, H3'), 4.48(m, 1H, H2'), 4.30(d, J=7.8 Hz, 1H, H20α), 4.25(m, 1H, H7), 4.16(d, J=7.8 Hz, 1H, H20β), 3.81(d, J= 7.2 Hz, 1H, H3), 3.34(d, J= 6.6 Hz, 1H, 2'OH), 2.55(m, 1H, H6α), 2.49(d, J=3.9 Hz, 1H, 7OH), 2.36(s, 3H, 4Ac), 2.32(m, 2H, H14), 2.27(s, 3H, 10Ac), 2.24(br s, 3H, Me18), 1.90(s, 3H, Me19), 1.86(m, 1H, H6β), 1.77(s, 3H, Me isobutenyl), 1.75(s, 1H, 1OH), 1.67(s, 3H, Me isobutenyl), 1.27(s, 3H, Me17), 1.16(s, 3H, Me16).

### EXAMPLE 10

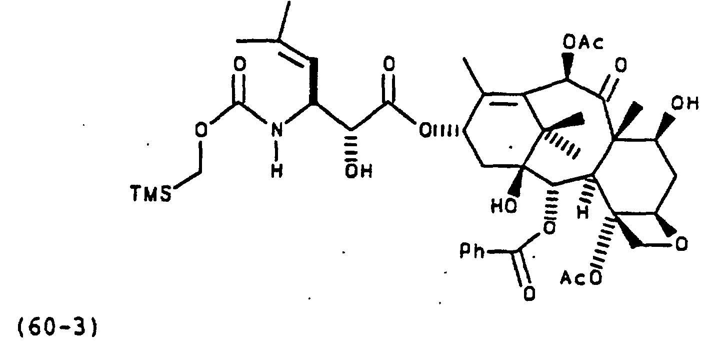

### Preparation of 3'-desphenyl-3'-isobutenyl-N-debenzoyl-N-(trimethylsilylmethoxycarbonyl) taxol.

To a solution of 7-triethylsilyl baccatin III (50.0 mg, 0.71 mmol) in 0.7 mL of THF at -45 °C was added dropwise 0.08 mL of a 1.0 M solution of LiN(SiMe₃)₂ in hexane. After 0.5 h at -45 °C, a solution of *cis*-1-(trimethylsilylmethoxycarbonyl)-3-(2-methoxy-2-propoxy)-4-(isobutenyl)azetidin-2-one (77.0 mg, 0.22 mmol) in 0.7 mL of THF was added dropwise to the mixture. The solution was warmed to 0°C and kept at that temperature for 1 h before 1.0 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 58.4 mg of a mixture containing (2'R,3'S)-2'-(2-methoxy-2-propoxy)-3'-desphenyl-3'-isobutenyl-7-triethylsilyl-N-debenzoyl-N-(trimethylsilylmethoxycarbonyl) taxol and a small amount of the (2'S,3'R) isomer.

To a solution of 58.4 mg (0.51 mmol) of the mixture obtained from the previous reaction in 5.0 mL of acetonitrile and 0.30 mL of pyridine at 0 °C was added 0.60 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 51.2 mg of material which was purified by flash chromatography to give 31.1 mg (71%) of 3'-desphenyl-3'-isobutenyl-N-debenzoyl-N-(trimethylsilylmethoxycarbonyl) taxol, which was recrystallized from methanol/water.

m.p.149-151°C; [α]²⁵_{Na} -58.0° (c 0.0018, CHCl₃).

¹H NMR (CDCl₃, 300 MHz) δ 8.11(d, J=7.2 Hz, 2H, benzoate ortho), 7.61(m, 1H, benzoate para), 7.48(m, 2H, benzoate meta), 6.30(s, 1H, H10), 6.21(dd, J = 7.5, 7.5 Hz, 1H, H13), 5.67(d, J = 7.2 Hz, 1H, H2β), 5.33(m, 1H, olefine of isobutenyl), 4.97(d, J= 7.8, 1H, H5), 4.88(d, J=8.2 Hz, 1H, NH), 4.76(ddd, J=8.7, 8.7, 2.7 Hz, 1H, H3'), 4.41(m, 1H, H2'), 4.28(d, J=7.8 Hz, 1H, H20α), 4.25(m, 1H, H7), 4.16(d, J=7.8 Hz, 1H, H20β), 3.76(d, J= 7.2 Hz, 1H, H3), 3.68(d, J=14.1 Hz, 1H, CH₂TMS), 3.51(d, J=14.1 Hz, 1H, CH₂TMS), 3.41(d, J= 6.6 Hz, 1H, 2'OH), 2.55(m, 1H, H6α), 2.50(d, J=3.9 Hz, 1H, 7OH) 2.29(s, 3H, 4Ac), 2.25(m, 2H, H14), 2.21(s, 3H, 10Ac), 2.24(br s, 3H, Me18), 1.89(s, 3H, Me19), 1.87(m, 1H, H6β), 1.77(s, 3H, Me isobutenyl), 1.75(s, 1H, 1OH), 1.68(s, 3H, Me isobutenyl), 1.18(s, 3H, Me17), 1.15(s, 3H, Me16), -0.04(s, 9H, Me₃Si-).

### EXAMPLE 11

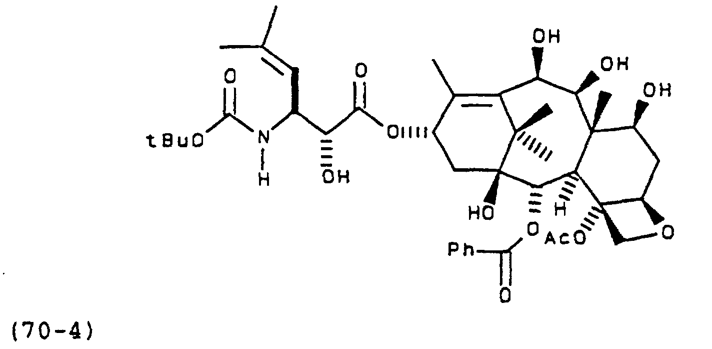

### Preparation of 3,-desphenyl-3'-(isobutenyl)-N-desbenzoyl-N-(t-butoxycarbonyl)-9-desoxo-9β-hydroxy-10-desacetyl taxol.

To a solution of 7,10-(bis)-O-triethylsilyl-9-desoxo-9β-hydroxy-10-deacetyl baccatin (III) (70.0 mg, 0.09 mmol) in 1.0 mL of THF at -45 °C was added dropwise 0.10 mL of a 0.98 M solution of LiN(SiMe₃)₂ in hexane. After 0.5 h at -45 °C, a solution of *cis*-1-(t-butoxycarbonyl)-3-(2-methoxyisopropyloxy)-4-(isobutenyl)-azetidin-2-one (84.5 mg, 0.27 mmol) in 1.0 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 88.3 mg of a mixture containing (2'R,3'S)-2',7,10-(tris)-O-triethylsilyl-3'-desphenyl-3'-(isobutenyl)-N-desbenzoyl-N-(t-butoxycarbonyl)-9-desoxo-9β-hydroxy-10-desacetyl taxol and a small amount of the (2'S,3'R) isomer.

To a solution of 88.3 mg (0.080 mmol) of the mixture obtained from the previous reaction in 13.5 mL of acetonitrile and 0.55 mL of pyridine at 0 °C was added 1.90 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 67.2 mg of material which was purified by flash chromatography to give 52.7 mg (82%) of 3'-desphenyl-3'-(isobutenyl)-N-desbenzoyl-N-(t-butoxycarbonyl)-9-desoxo-9β-hydroxy-10-desacetyl taxol, which was recrystallized from methanol/water.

m.p.138-140°C; [α]²⁵_{Na} -55.2° (c 0.0026, CHCl₃).

¹H NMR (MeOH, 300 MHz) δ 8.11(d, J=7.1 Hz, 2H, benzoate ortho), 7.61(m, 1H, benzoate para), 7.48(m, 2H, benzoate meta), 6.13(m, 1H, H13), 6.12(m, 1H, H2), 5.21(br s., 1H, H3'), 5.02(d, J=5.3 Hz, 1H, H10), 4.93(d, J=8.1 Hz, 1H, H5), 4.85(d, J=9.1 hz, 1H, NH), 4.84(d, J=8.5 Hz, 1H, Me₂C=CH-), 4.50(br s, 1H, H2'), 4.50(d, J=5.5 Hz, 1H, H9), 4.22(d, J=8.1, 1H, H20α), 4.18(d, J=8.1 Hz, 1H, H20β), 3.89(dd, J=9.4, 7.5 Hz, 1H, H7), 3.12(d, J=5.5 Hz, H3), 2.45(m, 1H, H6α), 2.31(m, 1H, H14α), 2.29(s, 3H, 4Ac), 2.18(m, 1H, H14β), 1.85(ddd, J=15.1, 9.4, 1.2 Hz, H6β), 1.81(s, 3H, Me16), 1.76(s, 3H, Me18), 1.72(s, 6H, 2Me from isobutenyl), 1.61(s, 3H, Me19), 1.39(s, 9H, 3Me t-butoxy), 1.26(s, 3H, Me17).

### EXAMPLE 12

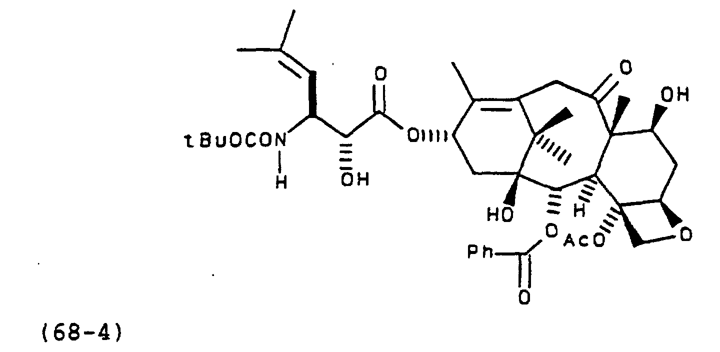

### Preparation of 3'-desphenyl-3'-(isobutenyl)-N-desbenzoyl-N-(t-butoxycarbonyl)-10-desacetoxy taxol.

To a solution of 7-0-triethylsilyl-10-desacetoxy baccatin (III) (50.0 mg, 0.077 mmol) in 0.8 mL of THF at -45°C was added dropwise 0.09 mL of a 0.98 M solution of LiN(SiMe₃)₂ in hexane. After 0.5 h at -45 °C, a solution of cis-1-t-butoxycarbonyl-3-(2-methoxyisopropyloxy)-4-(isobutenyl)azetidin-2-one (58.0 mg, 0.193 mmol) in 0.7 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 62.7 mg of a mixture containing (2'R,3'S)-2'-O-(2-methoxyisopropyl)-7-O-triethylsilyl-3'-desphenyl-3'-(isobutenyl)-N-desbenzoyl-N-(t-butoxycarbonyl)-10-desacetoxy taxol and a small amount of the (2'S,3'R) isomer.

To a solution of 62.7 mg (0.059 mmol) of the mixture obtained from the previous reaction in 3.5 mL of acetonitrile and 0.16 mL of pyridine at 0 °C was added 0.55 mL of 48% aqueous HF. The mixture was stirred at 0 °C for 3 h, then at 25°C for 13 h, and partitioned between saturated aqueous sodium bicarbonate and ethyl acetate. Evaporation of the ethyl acetate solution gave 51.5 mg of material which was purified by flash chromatography to give 43.0 mg (95%) of 3'-desphenyl-3'-(isobutenyl)-N-desbenzoyl-N-(t-butoxy-carbonyl)-10-desacetoxy taxol, which was recrystallized from methanol/water.

m.p.153-155°C; [α]²⁵_{Na} -56.3° (c 0.003, CHCl₃).

¹H NMR (CDCl₃, 300 MHz) δ 8.10(d, J=7.3 Hz, 2H, benzoate ortho), 7.60(m, 1H, benzoate para), 7.47(m, 2H, benzoate meta), 6.15(td, J=8.5, 1.8 Hz, 1H, H13), 5.69(d, J=6.9 Hz, 1H, H2), 5.32(d, J= 9.2 Hz, 1H, NH), 4.93(dd, J=9.6, 1.8 Hz, 1H, H5), 4.82(d, J=8.7 Hz, 1H, Me₂C=CH-), 4.76(td, J=8.7, 2.7 Hz, 1H, H3'), 4.37(d, J=8.7 Hz, 1H, H20α), 4.22(d, J=8.7 Hz, 1H, H20β), 4.18(d, J=2.7 Hz, 1H, H2'), 4.03(d, J=7.3 Hz, 1H, H7), 3.82(d, J=15.2 Hz, 1H, H10α), 3.47(m, 1H, 2'OH), 3.41(d, J=6.6 Hz, 1H, H3), 2.60(m, 1H, H6α), 2.39(m, 1H, H10β), 2.37(s, 3H, 4Ac), 2.18(s, 1H, 7 OH), 2.08(m, 1H, H14α), 1.78(m, 1H, H14β), 1.76(s, 3H, Me18), 1.74(s, 6H, 2Me from isobutenyl), 1.63(m, 1H, H6β), 1.36(s, 9H, 3Me t-butoxy) 1.26(s, 3H, Me17), 1.18(s, 3H, Me19), 1.15(s, 3H, Me16).

### EXAMPLE 13

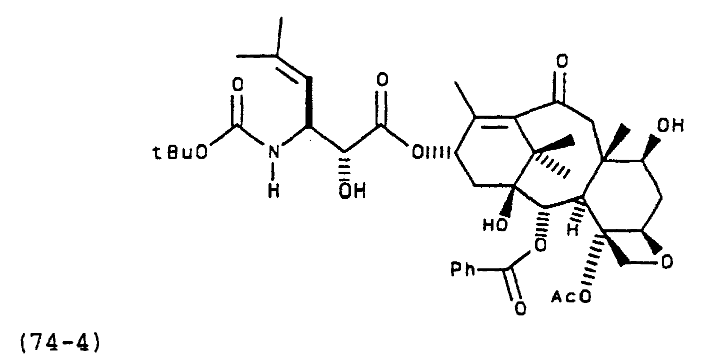

### Preparation of 3'-desphenyl-3'-(isobutenyl)-N-desbenzoyl-N-(t-butoxycarbonyl)-9-desoxo-10-desacetoxy-10-keto taxol.

To a solution of 7-O-triethylsilyl-9-desoxo-10-desacetoxy-10-keto baccatin (III) (30.0 mg, 0.047 mmol) in 0.5 mL of THF at -45 °C was added dropwise 0.05 mL of a 0.98 M solution of LiN(SiMe₃)₂ in hexane. After 0.5 h at -45 °C, a solution of *cis*-1-t-butoxycarbonyl-3-(2-methoxyisopropyloxy)-4-(isobutenyl) azetidin-2-one (44.1 mg, 0.141 mmol) in 0.5 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 40.8 mg of a mixture containing (2'R,3'S)-2'-O-(2-methoxyisopropyl)-7-0-triethylsilyl-3'-desphenyl-3'-(isobutenyl)-N-desbenzoyl-N-(t-butoxycarbonyl)-9-desoxo-10-desacetoxy-10-keto taxol and a small amount of the (2'S,3'R) isomer.

To a solution of 40.8 mg (0.043 mmol) of the mixture obtained from the previous reaction in 4 mL of acetonitrile and 0.2 mL of pyridine at 0 °C was added 0.5 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 34.4 mg of material which was purified by flash chromatography to give 23.0 mg (70%) of 3'-desphenyl-3'-(isobutenyl)-N-desbenzoyl-N-(t-butoxycarbonyl)-9-desoxo-10-desacetoxy-10-keto taxol, which was recrystallized from methanol/water.

m.p.149-153°C; [α]²⁵_{Na} -56.3° (c 0.0025, CHCl₃).

¹H NMR (CDCl₃, 300 MHz) δ 8.12(d, J=7.2 Hz, 2H, benzoate ortho), 7.64(m, 1H, benzoate para), 7.51(m, 2H, benzoate meta), 6.12(t, J=7.5 Hz, 1H, H13), 5.95(d, J=6.2 Hz, 1H, H2), 5.30(d, J=8.9 Hz, 1H, NH), 4.94(d, J=8.2 Hz, 1H, H5), 4.88(d, J=8.9 Hz, 1H, Me₂C=CH-), 4.79(td, J=8.9, 2.4 Hz, 1H, H3'), 4.34(d, J=8.2 Hz, 1H, H20α), 4.27(dd, J=5.5, 2.7 Hz, 1H, H2'), 4.19(d, J=8.2 Hz, 1H, H20β),, 3.73(m, 1H, H7), 3.67(br s, 1H, 2'OH), 3.13(d, J=5.1 Hz, 1H, H3), 3.12(d, J=15.7 Hz, 1H, H9α), 2.90(d, J=15.7 Hz, 1H, H9β), 2.55(m, 1H, H6α), 2.47(m, 1H, H14β), 2.32(s, 3H, 4Ac), 2.28(m, 1H, H14α), 2.04(br s, 1H, 7 OH), 1.88(s, 1H, 1 OH), 1.82(m, 1H, H6β), 1.79(s, 3H, Me18), 1.76(s, 6H, 2Me from isobutenyl), 1.57(s, 3H, Me16), 1.47 (s, 3H, Me19), 1.40(s, 9H, 3Me t-butoxy) 1.30(s, 3H, Me17).

### EXAMPLE 14

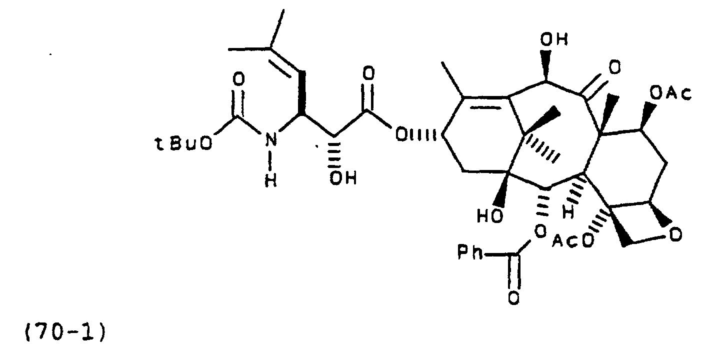

### Preparation of 3'-desphenyl-3'-(isobutenyl)-N-desbenzoyl-N-(t-butoxycarbonyl)-7-O-acetyl-10-desacetyl taxol.

To a solution of 7-O-triethylsilyl-9-desoxy-9β-acetoxy-10-desacetoxy-10-keto baccatin (III) (33.0 mg, 0.047 mmol) in 0.5 mL of THF at -45 °C was added dropwise 0.05 mL of a 0.98 M solution of LiN(SiMe₃)₂ in hexane. After 0.5 h at -45 °C, a solution of cis-1-t-butoxycarbonyl-3-(2-methoxyisopropyloxy)-4-isobutenylazetidin-2-one (44.1 mg, 0.141 mmol) in 0.5 mL of THF was added dropwise to the mixture. The solution was warmed to 0°C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 41.9 mg of a mixture containing (2'R,3'S)-2'-O-(2-methoxyisopropyl)-7-O-triethylsilyl-3'-desphenyl-3'-(isobutenyl)-N-desbenzoyl-N-(t-butoxycarbonyl)-9-desoxy-9β-acetoxy-10-desacetoxy-10-keto taxol and a small amount of the (2'S,3'R) isomer.

To a solution of 41.9 mg (0.045 mmol) of the mixture obtained from the previous reaction in 3.5 mL of acetonitrile and 0.15 mL of pyridine at 0 °C was added 0.50 mL of 48% aqueous HF. The mixture was stirred at 0°C for 3 h, then at 25 °C for 13 h, and partitioned between saturated aqueous sodium bicarbonate and ethyl acetate. Evaporation of the ethyl acetate solution gave 32.4 mg of material which was stirred with 1.0 g of silica gel in 5 mL of methylene chloride at room temperature in 48 hrs. The organic layer was purified by filtration through silica gel to give 26.2 mg (70%) of 3'-desphenyl-3'-(isobutenyl)-N-desbenzoyl-N-(t-butoxycarbonyl)-7-O-acetyl-10-desacetyl taxol.

m.p.1136-139°C; [α]²⁵_{Na} -60.2° (c 0.0025, CHCl₃).

¹H NMR (CDCl₃, 300 MHz) δ 8.10(d, J=7.3 Hz, 2H, benzoate ortho), 7.61(m, 1H, benzoate para), 7.48(m, 2H, benzoate meta), 6.16(td, J=8.7, 1.8 Hz, 1H, H13), 5.68(d, J=6.9 Hz, 1H, H2), 5.48(dd, J=10.5, 7.3 Hz, 1H, H7), 5.33(d, J=1.8 Hz, 1H, H10), 5.32(d, J= 9.2 Hz, 1H, NH), 4.94(dd, J=9.6, 1.8 Hz, 1H, H5), 4.80(d, J=8.7 Hz, 1H, Me₂C=CH-), 4.75(td, J=8.7, 2.7 Hz, 1H, H3'), 4.33(d, J=8.7 Hz, 1H, H20α), 4.23(d, J=2.7 Hz, 1H, H2'), 4.22(d, J=8.7 Hz, 1H, H20β), 4.01(d, J=6.9 Hz, 1H, H3), 3.98(d, J=1.8 Hz, 1H, 10OH), 3.68(m, 1H, 2'OH), 2.54(m, 1H, H6α), 2.37(s, 3H, 4Ac), 2.35(m, 1H, H14α), 2.01(m, 1H, H14β), 1.99(s, 3H, 7Ac), 1.98(br s, 3H, Me18), 1.93(m, 1H, H6β), 1.85(s, 3H, Me19), 1.77(s, 6H, 2Me from isobutenyl), 1.61(s, 1H, 7OH), 1.37(s, 9H, 3Me t-butoxy), 1.23(s, 3H, Me17), 1.10(s, 3H, Me16).

### EXAMPLE 15

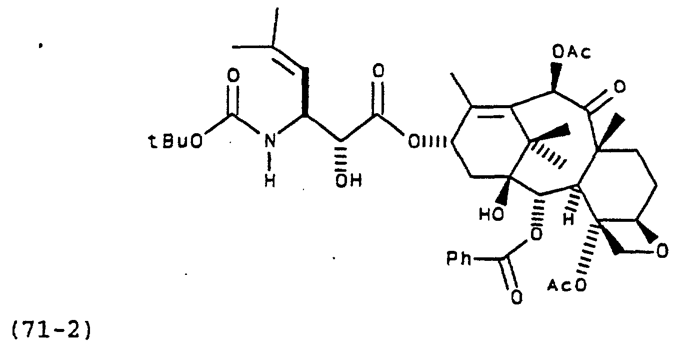

### Preparation of 3'-desphenyl-3'-(isobutenyl)-N-desbenzoyl-N-(t-butoxycarbonyl)-7-deshydroxy taxol.

To a solution of 7-deshydroxy baccatin (III) (38.7 mg, 0.063 mmol) in 0.8 mL of THF at -45 °C was added dropwise 0.08 mL of a 0.98 M solution of LiN(SiMe₃)₂ in hexane. After 0.5 h at -45 °C, a solution of *cis*-1-t-butoxycarbonyl-3-(2-methoxyisopropyloxy)-4-(isobutenyl)azetidin-2-one (59.0 mg, 0.19 mmol) in 0.8 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 43.4 mg of a mixture containing (2'R,3'S)-2'-O-(2-methoxyisopropyl)-3'-desphenyl-3'-(isobutenyl)-N-desbenzoyl-N-(t-butoxycarbonyl)-7-deshydroxy taxol and a small amount of the (2'S,3'R) isomer.

To a solution of 43.4 mg (0.049 mmol) of the mixture obtained from the previous reaction in 3.5 mL of acetonitrile and 0.15 mL of pyridine at 0 °C was added 0.5 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 40.2 mg of material which was purified by flash chromatography to give 34.1mg (86%) of 3'-desphenyl-3'-(isobutenyl)-N-desbenzoyl-N-(t-butoxycarbonyl)-7-deshydroxy taxol, which was recrystallized from methanol/water.

m.p.142-144°C; [α]²⁵_{Na} -53.3° (c 0.0024, CHCl₃).

¹H NMR (CDCl₃, 300 MHz) δ 8.13(d, J=7.3 Hz, 2H, benzoate ortho), 7.60(m, 1H, benzoate para), 7.47(m, 2H, benzoate meta), 6.41(s, 1H, H10), 6.20(dd, J=9.0, 0.9 Hz, 1H, H13), 5.67(d; J=7.2 Hz, 1H, H2), 5.39(d, J=6.9 Hz, 1H, NH), 5.32(d, J=9.0 Hz, 1H, H3'), 4.93(dd, J=8.7, 2.1 Hz, 1H, H5), 4.81(d, J=8.7 Hz, 1H, Me₂C=CH-), 4.61(d, J=3.3 Hz, 1H, H2'), 4.30(d, J=8.1 Hz, 1H, H20α), 4.17(d, J=8.1 Hz, 1H, H20β), 3.75(d, J=6.6 Hz, 1H, H3), 3.41(m, 1H, 2'OH), 2.36(s, 3H, 4Ac), 2.33(m, 1H, H14α), 2.30(m, 1H, H14β), 2.26(m, 1H, H6α), 2.08(m, 1H, H7α), 1.94(m, 1H, H6β), 1.85(br s, 3H, Me18), 1.73(s, 6H, 2Me from isobutenyl), 1.70(s, 3H, Me19), 1.66(s, 1H, 1 OH), 1.53(m, 1H, H7β), 1.41(s, 9H, 3Me t-butoxy), 1.25(s, 3H, Me16), 1.15(s, 3H, Me17).

### EXAMPLE 16

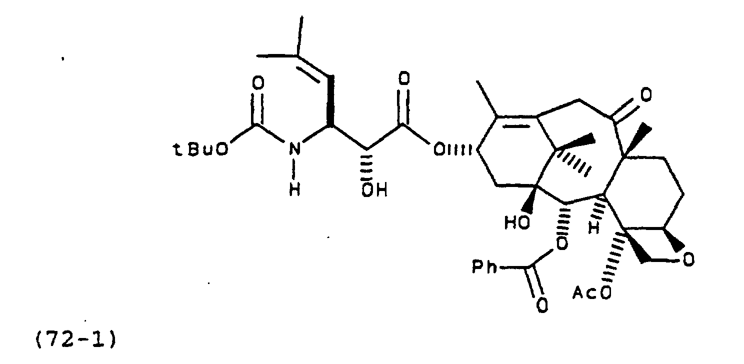

### Preparation of 3'-desphenyl-3'-(isobutenyl)-N-desbenzoyl-N-(t-butoxycarbonyl)-7-deshydroxy-10-desacetoxy taxol.

To a solution of 7-deshydroxy-10-desacetoxy baccatin (III) (28.7 mg, 0.051 mmol) in 0.7 mL of THF at -45°C was added dropwise 0.06 mL of a 0.98 M solution of LiN(SiMe₃)₂ in hexane. After 0.5 h at -45 °C, a solution of *cis*-1-t-butoxycarbonyl-3-(2-methoxyisopropyloxy)-4-(isobutenyl)azetidin-2-one (47.3 mg, 0.15 mmol) in 0.7 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 40.3 mg of a mixture containing (2'R,3'S)-2'-O-(2-methoxyisopropyl)-3'-desphenyl-3'-(isobutenyl)-N-debenzoyl-N-(t-butoxycarbonyl)-7-deshydroxy-10-desacetoxy taxol and a small amount of the (2'S,3'R) isomer.

To a solution of 40.3 mg (0.046 mmol) of the mixture obtained from the previous reaction in 3.2 mL of acetonitrile and 0.15 mL of pyridine at 0 °C was added 0.47 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 35.2 mg of material which was purified by flash chromatography to give 24.0 mg (70%) of 3'-desphenyl-3'-(isobutenyl)-N-debenzoyl-N-(t-butoxycarbonyl)-7-deshydroxy-10-desacetoxy taxol, which was recrystallized from methanol/water.

m.p.122-125°C; [α]²⁵_{Na} -64.3° (c 0.0025, CHCl₃).

¹H NMR (CDCl₃, 300 MHz) δ 8.12(d, J=7.1 Hz, 2H, benzoate ortho), 7.60(m, 1H, benzoate para), 7.48(m, 2H, benzoate meta), 6.11(td, J=8.1, 1.8 Hz, 1H, H13), 5.68(d, J=6.9 Hz, 1H, H2), 5.23(d, J=9.9 Hz, 1H, NH), 5.12(d, J=9.9 Hz, 1H, H3'), 4.96(dd, J=9.1, 2.7 Hz, 1H, H5), 4.80(d, J=8.7 Hz, 1H, Me₂C=CH-), 4.58(dd, J=5.7, 2.1 Hz, 1H, H2'), 4.30(d, J=8.1, 1H, H20α), 4.19(d, J=8.1 Hz, 1H, H20β), 3.97(d, J=6.9 Hz, H3), 3.83(d, J=16.5, 1H, H10α), 3.33(m, 1H, H10β), 3.30(m, 1H, 2'OH), 2.39(m, 1H, H14α), 2.35(s, 3H, 4Ac), 2.26(m, 1H, H14β), 2.19(m, 1H, H6α), 2.10(m, 1H, H7α), 1.95(m, 1H, H6β), 1.73(s, 3H, Me18), 1.69(s, 6H, 2Me from isobutenyl), 1.63(s, 3H, Me19), 1.44(m, 1H, H7β), 1.39(br. s, 1H, 1 OH), 1.35(s, 9H, 3Me t-butoxy), 1.25(s, 3H, Me16), 1.15(s, 3H, Me17).

### EXAMPLE 17

Taxanes 29-4, 51-3, 54-1, 54-2, 54-3, 58-3, 58-4, 59-1, 59-2, 60-3, 70-4, 68-4, 74-4, 70-1, 71-2, and 72-1 of Examples 1 - 16 were evaluated in in vitro cytotoxicity activity against human colon carcinoma cells HCT-116. Cytotoxicity was assessed in HCT116 human colon carcinoma cells by XTT (2,3-bis(2-methoxy-4-nitro-5-sulfophenyl)-5-[(phenylamino)carbonyl]-2H-tetrazolium hydroxide) assay (Scudiero et al, "Evaluation of a soluble tetrazolium/formazan assay for cell growth and drug sensitivity in culture using human and other tumor cell lines", Cancer Res. 48:4827-4833, 1988). Cells were plated at 4000 cells/well in 96 well microtiter plates and 24 hours later drugs were added and serial diluted. The cells were incubated at 37°C for 72 hours at which time the tetrazolium dye, XTT, was added. A dehydrogenase enzyme in live cells reduces the XTT to a form that absorbs light at 450 nm which can be quantitated spectrophotometrically. The greater the absorbance the greater the number of live cells. The results are expressed as an IC₅₀ which is the drug concentration required to inhibit cell proliferation (i.e. absorbance at 450 nm) to 50% of that of untreated control cells.

All compounds had an IC₅₀ of less than 0.1, indicating that they are cytotoxically active.

## Claims

1. A taxane derivative having the formula 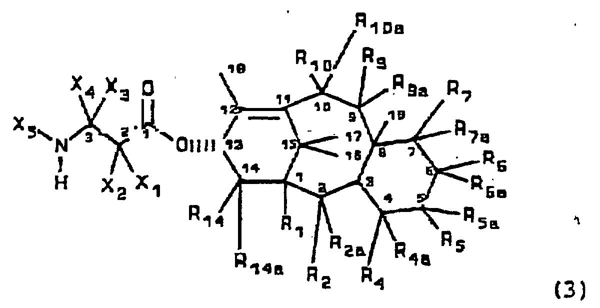 wherein
X₁ is -OX₆, -SX₇, or -NX₈X₉;
X₂ is hydrogen, alkyl, alkenyl, alkynyl, aryl, or heteroaryl;
X₃ is hydrogen;
X₄ is isobutenyl;
X₅ is -COX₁₀, -COOX₁₀, -COSX₁₀, -CONX₈X₁₀, or -SO₂X₁₁;
X₆ is hydrogen, alkyl, alkenyl, alkynyl, aryl, or heteroaryl;
X₇ is alkyl, alkenyl, alkynyl, aryl, heteroaryl, or a sulfhydryl protecting group;
X₈ is hydrogen, alkyl, alkenyl, alkynyl, aryl, heteroaryl, or heterosubstituted alkyl, alkenyl, alkynyl, ary or heteroaryl;
X₉ is an amino protecting group;
X₁₀ is alkyl, alkenyl, alkynyl, aryl, heteroaryl, or heterosubstituted alkyl, alkenyl alkynyl, aryl or heteroaryl;
X₁₁ is alkyl, alkenyl, alkynyl, aryl, heteroaryl, -OX₁₀, or -NX₈X₁₄;
X₁₄ is hydrogen, alkyl, alkenyl, alkynyl, aryl, or heteroaryl;
R₁ is hydrogen, hydroxy, protected hydroxy or together with R₁₄ forms a carbonate;
R₂ is hydrogen, hydroxy, -OCOR₃₁ together with R₂ₐ forms an oxo;
R₂ₐ is hydrogen or taken together with R₂ forms an oxo or;
R₄ is hydrogen, together with R₄ₐ forms an oxo, oxirane or methylene, or together with R₅ₐ and the carbon atoms to which they are attached form an oxetane ring;
R₄ₐ is hydrogen, alkyl, alkenyl, alkynyl, aryl, heteroaryl, cyano, hydroxy, -OCOR₃₀, or together with R₄ forms an oxo, oxirane or methylene;
R₅ is hydrogen or together with R₅ₐ forms an oxo;
R₅ₐ is hydrogen, hydroxy, protected hydroxy, acyloxy, together with R₅ forms an oxo, or together with R₄ and the carbon atoms to which they are attached form an oxetane ring;
R₆ is hydrogen, alkyl, alkenyl, alkynyl, aryl, or heteroaryl, hydroxy, protected hydroxy or together with R₆ₐ forms an oxo;
R₆ₐ is hydrogen, alkyl, alkenyl, alkynyl, aryl, or heteroaryl, hydroxy, protected hydroxy or together with R₆ forms an oxo;
R₇ is hydrogen or together with R₇ₐ forms an oxo;
R₇ₐ is hydrogen, halogen, protected hydroxy, -OR₂₈, or together with R₇ forms an oxo;
R₉ is hydrogen or together with R₉ₐ forms an oxo;
R₉ₐ is hydrogen, hydroxy, protected hydroxy, acyloxy, or together with R₉ forms an oxo;
R₁₀ is hydrogen or together with R₁₀ₐ forms an oxo;
R₁₀ₐ is hydrogen, -OCOR₂₉, hydroxy, or protected hydroxy, or together with R₁₀ forms an oxo;
R₁₄ is hydrogen, alkyl, alkenyl, alkynyl, aryl, heteroaryl, hydroxy, a protected hydroxy or together with R₁ forms a carbonate;
R₁₄ₐ is hydrogen, alkyl, alkenyl, alkynyl, aryl, or heteroaryl;
R₂₈ is hydrogen, acyl, or a hydroxy protecting group; and
R₂₉, R₃₀, and R₃₁ are independently hydrogen, alkyl, alkenyl, alkynyl, monocyclic aryl or monocyclic heteroaryl.

2. The taxane derivative of claim 1 wherein X₅ is -COOX₁₀ and X₁₀ is alkyl.

3. The taxane derivative of claim 1 wherein
R₁₄ and R₁₄ₐ are hydrogen;
R₁₀ is hydrogen or together with R₁₀ₐ forms an oxo;
R₁₀ₐ is hydrogen, hydroxy, -OCOR₂₉, or together with R₁₀ forms an oxo;
R₉ is hydrogen or together with R₉ₐ forms an oxo;
R₉ₐ is hydrogen, acyloxy or together with R₉ forms an oxo;
R₇ is hydrogen;
R₇ₐ is hydrogen, halogen or -OR₂₈;
R₆ and R₆ₐ are hydrogen;
R₅ is hydrogen;
R₅ₐ together with R₄ and the carbon atoms to which they are attached form an oxetane ring;
R₄ₐ is hydroxy, or -OCOR₃₀;
R₂ is hydroxy, or -OCOR₃₁;
R₂ₐ is hydrogen;
R₁ is hydrogen or hydroxy;
R₂₈ is hydrogen, acyl or hydroxy protecting group; and
R₂₉, R₃₀, and R₃₁ are independently hydrogen, alkyl, alkenyl, alkynyl, monocyclic aryl or monocyclic heteroaryl.

4. The taxane derivative of claim 2 wherein
R₁₄ and R₁₄ₐ are hydrogen;
R₁₀ is hydrogen or together with R₁₀ₐ forms an oxo;
R₁₀ₐ is hydrogen, hydroxy, -OCOR₂₉, or together with R₁₀ forms an oxo;
R₉ is hydrogen or together with R₉ₐ forms an oxo;
R₉ₐ is hydrogen, acyloxy or together with R₉ forms an oxo;
R₇ is hydrogen;
R₇ₐ is hydrogen, halogen or -OR₂₈;
R₆ and R₆ₐ are hydrogen;
R₅ is hydrogen;
R₅ₐ together with R₄ and the carbon atoms to which they are attached form an oxetane ring;
R₄ₐ is hydroxy, or -OCOR₃₀;
R₂ is hydroxy, or -OCOR₃₁;
R₂ₐ is hydrogen;
R₁ is hydrogen or hydroxy;
R₂₈ is hydrogen, acyl or hydroxy protecting group; and
R₂₉, R₃₀, and R₃₁ are independently hydrogen, alkyl, alkenyl, alkynyl, monocyclic aryl or monocyclic heteroaryl.

5. A taxane according to claim 1, wherein R₁₄ and R₁₄ₐ are hydrogen, R₁₀ is hydrogen, R₁₀ₐ is hydroxy or acetoxy, R₉ and R₉ₐ together form an oxo, R₇ is hydrogen, R₇ₐ is hydroxy, R₅ is hydrogen, R₅ₐ and R₄ and the carbons to which they are attached form an oxetane ring, R₄ₐ is acetoxy, R₂ is hydrogen, R₂ₐ is benzoyloxy, and R₁ is hydroxy.

6. A taxane according to claim 1, wherein R₁₄ is hydroxy, R₂ is hydroxy or -OCOR₃₁ wherein R₃₁ is hydrogen, alkyl or selected from the group consisting comprising 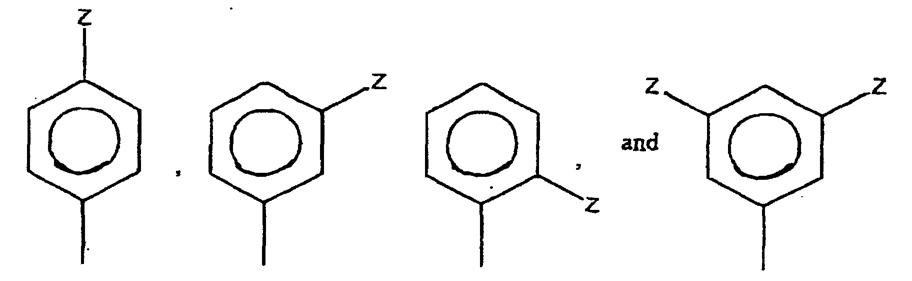 and Z is alkyl, hydroxy, alkoxy, halogen, or trifluoromethyl.

7. A taxane according to claim 1 wherein R₉ₐ is hydrogen and R₉ is hydrogen or hydroxy, R₇ₐ is hydrogen and R₇ is acetoxy or other acyloxy or halogen, or R₁₀ and R10a are each hydrogen or together form an oxo.

8. A taxane according to claim 1, wherein X₁ is -OH, X₂ is hydrogen, X₃ is hydrogen, X₄ is isobutenyl, X₅ is -COX₁₀ wherein X₁₀ is furyl, thienyl, alkyl substituted furyl or thienyl, or X₅ is -COOX₁₀ wherein X₁₀ is t-butyl, isobutyl, or n-butyl, ethyl, isopropyl, n-propyl, cyclohexyl, allyl, crotyl, 1,3-diethoxy-2-propyl, 2-methoxyethyl, amyl, neopentyl, or PhCH₂-, or X₅ is -NX₈X₁₀ wherein X₈ and X₁₀ are both phenyl, or X₈ is hydrogen and X₁₀ is n-propyl, phenyl or ethyl.

9. A taxane derivative having the formula 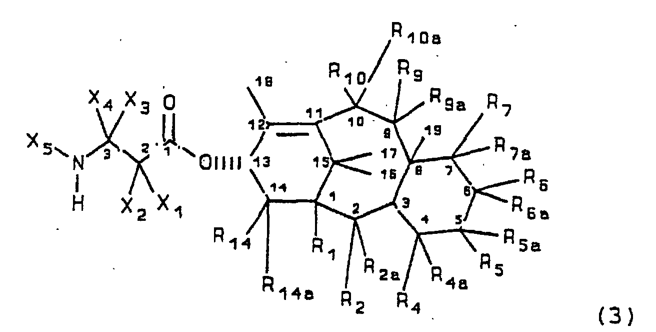 wherein
X₁ is -OX₆;
X₂ is hydrogen;
X₃ is hydrogen;
X₄ is isobutenyl;
X₅ is -COX₁₀, -COOX₁₀, -COSX₁₀, -CONX₈X₁₀, or -SO₂X₁₁;
X₆ is hydrogen;
X₈ is hydrogen, alkyl, alkenyl, alkynyl, aryl, heteroaryl, or heterosubstituted alkyl, alkenyl, alkynyl, aryl or heteroaryl;
X₉ is an amino protecting group;
X₁₀ is alkyl, alkenyl, alkynyl, aryl, heteroaryl, or heterosubstituted alkyl, alkenyl, alkynyl, aryl or heteroaryl;
X₁₁ is alkyl, alkenyl, alkynyl, aryl, heteroaryl, -OX₁₀, or -NX₈X₁₄;
X₁₄ is hydrogen, alkyl, alkenyl, alkynyl, aryl, or heteroaryl;
R₁ is hydrogen, hydroxy, protected hydroxy or together with R₁₄ forms a carbonate;
R₂ is hydrogen, hydroxy, -OCOR₃₁ together with R₂ₐ forms an oxo;
R₂ₐ is hydrogen or taken together with R₂ forms an oxo or;
R₄ is hydrogen, together with R₄ₐ forms an oxo, oxirane or methylene, or together with R₅ₐ and the carbon atoms to which they are attached form an oxetane ring;
R₄ₐ is hydrogen, alkyl, alkenyl, alkynyl, aryl, heteroaryl, cyano, hydroxy, -OCOR₃₀, or together with R₄ forms an oxo, oxirane or methylene;
R₅ is hydrogen or together with R₅ₐ forms an oxo;
R₅ₐ is hydrogen, hydroxy, protected hydroxy, acyloxy, together with R₅ forms an oxo, or together with R₄ and the carbon atoms to which they are attached form an oxetane ring;
R₆ is hydrogen, alkyl, alkenyl, alkynyl, aryl, or heteroaryl, hydroxy, protected hydroxy or together with R₆ₐ forms an oxo;
R₆ₐ is hydrogen, alkyl, alkenyl, alkynyl, aryl, or heteroaryl, hydroxy, protected hydroxy or together with R₆ forms an oxo;
R₇ is hydrogen or together with R₇ₐ forms an oxo;
R₇ₐ is hydrogen, halogen, protected hydroxy, -OR₂₈, or together with R₇ forms an oxo;
R₉ is hydrogen or together with R₉ₐ forms an oxo;
R₉ₐ is hydrogen, hydroxy, protected hydroxy, acyloxy, or together with R₉ forms an oxo;
R₁₀ is hydrogen or together with R₁₀ₐ forms an oxo;
R₁₀ₐ is hydrogen, -OCOR₂₉, hydroxy, or protected hydroxy, or together with R₁₀ forms an oxo;
R₁₄ is hydrogen, hydroxy, protected hydroxy or together with R₁ forms a carbonate;
R₁₄ₐ is hydrogen;
R₂₈ is hydrogen, acyl, or a hydroxy protecting group; and
R_{29,} R₃₀, and R₃₁ are independently hydrogen, alkyl, alkenyl, alkynyl, monocyclic aryl or monocyclic heteroaryl.

10. The taxane derivative of claim 9 wherein X₅ is -COX₁₀ wherein X₁₀ is furyl, thienyl, alkyl substituted furyl or thienyl or X₅ is -COOX₁₀ wherein X₁₀ is t-butyl, isobutyl, or n-butyl, ethyl, isopropyl, n-propyl, cyclohexyl, allyl, crotyl, 1,3-diethoxy-2-propyl, 2-methoxyethyl, amyl, neopentyl, or PhCH₂-, or X₅ is -CONX₈X₁₀ wherein X₈ and X₁₀ are both phenyl, or X₈ is hydrogen and X₁₀ is n-propyl, phenyl or ethyl.

11. A β-lactam of the formula 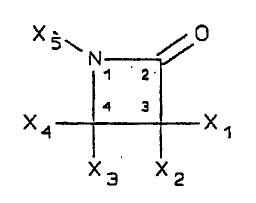 wherein
X₁ is -OX₆, -SX₇, or -NX₈X₉;
X₂ is hydrogen, alkyl, alkenyl, alkynyl, aryl, or heteroaryl;
X₃ is hydrogen;
X₄ is isobutenyl;
X₅ is -COX₁₃, -COOX₁₀, -COSX₁₂, -CONX₈X₁₂, or -SO₂X₁₁;
X₆ is hydrogen, alkyl, alkenyl, alkynyl, aryl, or heteroaryl;
X₇ is alkyl, alkenyl, alkynyl, aryl, heteroaryl, or a sulfhydryl protecting group;
X₈ is hydrogen, alkyl, alkenyl, alkynyl, aryl, heteroaryl, or heterosubstituted alkyl, alkenyl, alkynyl, aryl or heteroaryl;
X₉ is an amino protecting group;
X₁₀ is alkenyl, alkynyl, aryl, heteroaryl, or heterosubstituted alkyl, alkenyl, alkynyl, aryl or heteroaryl;
X₁₁ is alkyl, alkenyl, alkynyl, aryl, hereroaryl, -OX₁₀, or -NX₈X₁₄;
X₁₂ is alkyl, alkenyl, alkynyl, aryl, heteroaryl, or heterosubstituted alkyl, alkenyl, alkynyl, aryl or heteroaryl;
X₁₃ is alkenyl, alkynyl, heteroaryl, or heterosubstituted alkyl, alkenyl, alkynyl or heteroaryl;
X₁₄ is hydrogen, alkyl, alkenyl, alkynyl, aryl, or heteroaryl.

12. A β-lactam of the formula 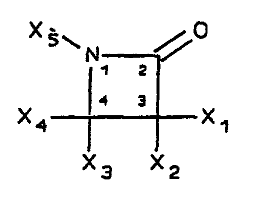 wherein
X₁ is -OX₆, -SX₇, or -NX₈X₉;
X₂ is alkenyl, alkynyl, aryl, or heteroaryl;
X₃ is hydrogen;
X₄ is isobutenyl
X₅ is -COX₁₀, -COOX₁₀, -COSX₁₀, -CONX₈X₁₀, or -SO₂X₁₁;
X₆ is hydrogen, alkyl, alkenyl, alkynyl, aryl, or heteroaryl;
X₇ is alkyl, alkenyl, alkynyl, aryl, heteroaryl, or a sulfhydryl protecting group;
X₈ is hydrogen, alkyl, alkenyl, alkynyl, aryl, heteroaryl, or heterosubstituted alkyl, alkenyl, alkynyl, aryl or heteroaryl;
X₉ is an amino protecting group;
X₁₀ is alkyl, alkenyl, alkynyl, aryl, heteroaryl, or heterosubstituted alkyl, alkenyl, alkynyl, aryl or heteroaryl;
X₁₁ is alkyl, alkenyl, alkynyl, aryl, heteroaryl, -OX₁₀, or -NX₈X₁₄;
X₁₄ is hydrogen, alkyl, alkenyl, alkynyl, aryl, or heteroaryl.

## Patentansprüche

1. Taxanderivat mit der Formel 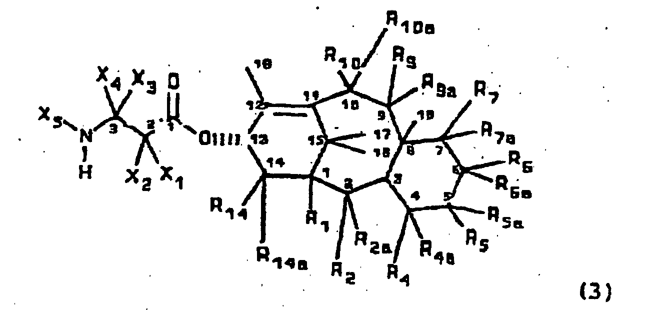 worin
X₁ -OX₆, -SX₇ oder -NX₈X₉ ist ;
X₂ Wasserstoff, Alkyl, Alkenyl, Alkynyl, Aryl oder Heteroaryl ist;
X₃ Wasserstoff ist;
X₄ Isobutenyl ist;
X₅ -COX₁₀, -COOX₁₀, -COSX₁₀, -CONX₈X₁₀ oder -SO₂X₁₁ ist;
X₆ Wasserstoff, Alkyl, Alkenyl, Alkynyl, Aryl oder Heteroaryl ist;
X₇ Alkyl, Alkenyl, Alkynyl, Aryl, Heteroaryl oder eine Sulfhydrylschutzgruppe ist;
X₈ Wasserstoff, Alkyl, Alkenyl, Alkynyl, Aryl, Heteroaryl oder heterosubstituiertes Alkyl, Alkenyl, Alkynyl, Aryl oder Heteroaryl ist;
X₉ eine Amino-Schutzgruppe ist;
X₁₀ Alkyl, Alkenyl, Alkynyl, Aryl, Heteroaryl oder heterosubstituiertes Alkyl, Alkenyl, Alkynyl, Aryl oder Heteroaryl ist;
X₁₁ Alkyl, Alkenyl, Alkynyl, Aryl, Heteroaryl, -OX₁₀ oder -NX₈X₁₄ ist;
X₁₄ Wasserstoff, Alkyl, Alkenyl, Alkynyl, Aryl oder Heteroaryl ist;
R₁ Wasserstoff, Hydroxy, geschütztes Hydroxy ist oder zusammen mit R₁₄ ein Carbonat bildet;
R₂ Wasserstoff, Hydroxy, -OCOR₃₁ ist oder zusammen mit R₂ₐ ein Oxo bildet;
R₂ₐ Wasserstoff ist oder zusammen mit R₂ ein Oxo bildet;
R₄ Wasserstoff ist, zusammen mit R₄ₐ ein Oxo, Oxiran oder Methylen bildet oder zusammen mit R₅ₐ und den Kohlenstoffatomen, an denen sie hängen, einen Oxetanring bildet;
R₄ₐ Wasserstoff, Alkyl, Alkenyl, Alkynyl, Aryl, Heteroaryl, Cyano, Hydroxy, -OCOR₃₀ ist oder zusammen mit R₄ ein Oxo, Oxiran oder Methylen bildet;
R₅ Wasserstoff ist oder zusammen mit R₅ₐ ein Oxo bildet;
R₅ₐ Wasserstoff, Hydroxy, geschütztes Hydroxy, Acyloxy ist, zusammen mit R₅ ein Oxo bildet oder zusammen mit R₄ und den Kohlenstoffatomen, an denen sie hängen, einen Oxetanring bildet;
R₆ Wasserstoff, Alkyl, Alkenyl, Alkynyl, Aryl oder Heteroaryl, Hydroxy, geschütztes Hydroxy ist oder zusammen mit R₆ₐ ein Oxo bildet;
R₆ₐ Wasserstoff, Alkyl, Alkenyl, Alkynyl, Aryl oder Heteroaryl, Hydroxy, geschütztes Hydroxy ist oder zusammen mit R₆ ein Oxo bildet;
R₇ Wasserstoff ist oder zusammen mit R₇ₐ ein Oxo bildet;
R₇ₐ Wasserstoff, Halogen, geschütztes Hydroxy, -OR₂₈ ist oder zusammen mit R₇ ein Oxo bildet;
R₉ Wasserstoff ist oder zusammen mit R₉ₐ ein Oxo bildet;
R₉ₐ Wasserstoff, Hydroxy, geschütztes Hydroxy, Acyloxy ist oder zusammen mit R₉ ein Oxo bildet;
R₁₀ Wasserstoff ist oder zusammen mit R₁₀ₐ ein Oxo bildet;
R₁₀ₐ Wasserstoff, -OCOR₂₉, Hydroxy oder geschütztes Hydroxy ist oder zusammen mit R₁₀ ein Oxo bildet;
R₁₄ Wasserstoff, Alkyl, Alkenyl, Alkynyl, Aryl, Heteroaryl, Hydroxy, ein geschütztes Hydroxy ist oder zusammen mit R₁ ein Carbonat bildet;
R₁₄ₐ Wasserstoff, Alkyl, Alkenyl, Alkynyl, Aryl oder Heteroaryl ist;
R₂₈ Wasserstoff, Acyl oder eine Hydroxy-Schutzgruppe ist; und
R₂₉ , R₃₀ und R₃₁ unabhängig Wasserstoff, Alkyl, Alkenyl, Alkynyl, monocyclisches Aryl oder monocyclisches Heteroaryl sind.

2. Taxanderivat des Anspruchs 1, bei dem X₅ -COOX₁₀ ist und X₁₀ Alkyl ist.

3. Taxanderivat des Anspruchs 1, bei dem
R₁₄ und R₁₄ₐ Wasserstoff sind;
R₁₀ Wasserstoff ist oder zusammen mit R₁₀ₐ ein Oxo bildet;
R₁₀ₐ Wasserstoff, Hydroxy, -OCOR₂₉ ist oder zusammen mit R₁₀ ein Oxo bildet;
R₉ Wasserstoff ist oder zusammen mit R₉ₐ ein Oxo bildet;
R₉ₐ Wasserstoff, Acyloxy ist oder zusammen mit R₉ ein Oxo bildet;
R₇ Wasserstoff ist;
R₇ₐ Wasserstoff, Halogen oder -OR₂₈ ist;
R₆ und R₆ₐ Wasserstoff sind;
R₅ Wasserstoff ist;
R₅ₐ zusammen mit R₄ und den Kohlenstoffatomen, an denen sie hängen, einen Oxetanring bildet;
R₄ₐ Hydroxy oder -OCOR₃₀ ist;
R₂ Hydroxy oder -OCOR₃₁ ist;
R₂ₐ Wasserstoff ist;
R₁ Wasserstoff oder Hydroxy ist;
R₂₈ Wasserstoff, Acyl oder eine Hydroxy-Schutzgruppe ist; und
R₂₉ , R₃₀ und R₃₁ unabhängig Wasserstoff, Alkyl, Alkenyl, Alkynyl, monocyclisches Aryl oder monocyclisches Heteroaryl sind.

4. Taxanderivat des Anspruchs 2, bei dem
R₁₄ und R₁₄ₐ Wasserstoff sind;
R₁₀ Wasserstoff ist oder zusammen mit R₁₀ₐ ein Oxo bildet;
R₁₀ₐ Wasserstoff, Hydroxy oder -OCOR₂₉ ist oder zusammen mit R₁₀ ein Oxo bildet;
R₉ Wasserstoff ist oder zusammen mit R₉ₐ ein Oxo bildet;
R₉ₐ Wasserstoff, Acyloxy ist oder zusammen mit R₉ ein Oxo bildet;
R₇ Wasserstoff ist;
R₇ₐ Wasserstoff, Halogen oder -OR₂₈ ist;
R₆ und R₆ₐ Wasserstoff sind;
R₅ Wasserstoff ist;
R₅ₐ zusammen mit R₄ und den Kohlenstoffatomen, an denen sie hängen, einen Oxetanring bildet;
R₄ₐ Hydroxy oder -OCOR₃₀ ist;
R₂ Hydroxy oder -OCOR₃₁ ist;
R₂ₐ Wasserstoff ist;
R₁ Wasserstoff oder Hydroxy ist;
R₂₈ Wasserstoff, Acyl oder Hydroxy-Schutzgruppe ist; und
R₂₉ , R₃₀ und R₃₁ unabhängig Wasserstoff, Alkyl, Alkenyl, Alkynyl, monocyclisches Aryl oder monocyclisches Heteroaryl sind.

5. Taxan gemäß Anspruch 1, bei dem R₁₄ und R₁₄ₐ Wasserstoff sind, R₁₀ Wasserstoff ist, R₁₀ₐ Hydroxy oder Acetoxy ist, R₉ₐ und R₉ₐ zusammen mit ein Oxo bilden, R₇ Wasserstoff ist, R₇ₐ Hydroxy ist, R₅ Wasserstoff ist; R₅ₐ und R₄ und die Kohlenstoffatome, an denen sie hängen, einen Oxetanring bilden, R₄ₐ Acetoxy ist, R₂ Wasserstoff ist, R₂ₐ Benzoyloxy ist, und R₁ Hydroxy ist.

6. Taxan gemäß Anspruch 1, bei dem R₁₄ Hydroxy ist, R₂ Hydroxy oder -OCOR₃₁ ist, worin R₃₁ Wasserstoff oder Alkyl ist oder aus der aus 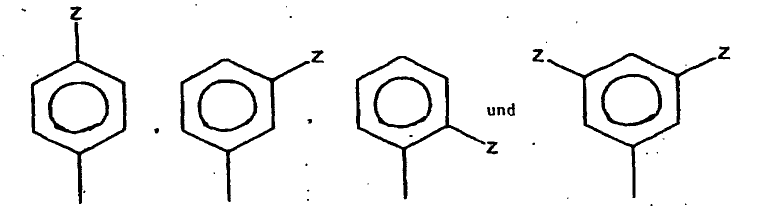 bestehenden Gruppe ausgewählt ist und Z Alkyl, Hydroxy, Alkoxy, Halogen oder Trifluormethyl ist.

7. Taxan gemäß Anspruch 1, bei dem R₉ₐ Wassertsoff ist und R₅ Wasserstoff oder Hydroxy ist, R₇ₐ Wasserstoff ist und R₇ Acetoxy ist oder anderes Acyloxy oder Halogen ist oder R₁₀ und R₁₀ₐ jeweils Wasserstoff sind oder zusammen ein Oxo bilden.

8. Taxan gemäß Anspruch 1, bei dem X₁ -OH ist, X₂ Wasserstoff ist, X₃ Wasserstoff ist, X₄ iso-Butenyl ist, X₅ -COX₁₀ ist, worin X₁₀ Furyl, Thienyl, alkylsubstituiertes Furyl oder Thienyl ist oder X₅ -COOX₁₀ ist, worin X₁₀ t-Butyl, iso-Butyl oder n-Butyl, Ethyl, iso-Propyl, n-Propyl, Cyclohexyl, Allyl, Crotyl, 1,3-Diethoxy-2-propyl, 2-Methoxyethyl, Amyl, Neopentyl oder PhCH₂- ist oder X₅ -NX₈X₁₀ ist, worin X₈ und X₁₀ beide Phenyl sind oder X₈ Wasserstoff ist und X₁₀ n-Propyl, Phenyl oder Ethyl ist.

9. Taxanderivat mit der Formel 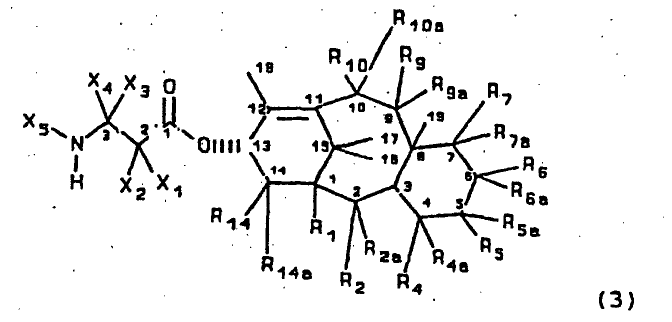 worin
X₁ -OX₆ ist;
X₂ Wasserstoff ist;
X₃ Wasserstoff ist;
X₄ Isobutenyl ist;
X₅ -COX₁₀ , -COOX₁₀ , -COSX₁₀ , -CONX₈X₁₀ oder -SO₂X₁₁ ist;
X₆ Wasserstoff ist;
X₈ Wasserstoff, Alkyl, Alkenyl, Alkynyl, Aryl, Heteroaryl oder heterosubstituiertes Alkyl, Alkenyl, Alkynyl, Aryl oder Heteroaryl ist;
X₉ eine Amino-Schutzgruppe ist;
X₁₀ Alkyl, Alkenyl, Alkynyl, Aryl, Heteroaryl oder heterosubstituiertes Alkyl, Alkenyl, Alkynyl, Aryl oder Heteroaryl ist;
X₁₁ Alkyl, Alkenyl, Alkynyl, Aryl, Heteroaryl, -OX₁₀ oder -NX₈X₁₄ ist;
X₁₄ Wasserstoff, Alkyl, Alkenyl, Alkynyl, Aryl oder Heteroaryl ist;
R₁ Wasserstoff, Hydroxy, geschütztes Hydroxy ist oder zusammen mit R₁₄ ein Carbonat bildet;
R₂ Wasserstoff, Hydroxy oder -OCOR₃₁ ist oder zusammen mit R₂ₐ ein Oxo bildet;
R₂ₐ Wasserstoff ist oder zusammen mit R₂ ein Oxo bildet;
R₄ Wasserstoff ist, zusammen mit R₄ₐ ein Oxo, Oxiran oder Methylen bildet oder zusammen mit R₅ₐ und den Kohlenstoffatomen, an denen sie hängen, einen Oxetanring bildet;
R₄ₐ Wasserstoff, Alkyl, Alkenyl, Alkynyl, Aryl, Heteroaryl, Cyano, Hydroxy, -OCOR₃₀ ist oder zusammen mit R₄ ein Oxo, Oxiran oder Methylen bildet;
R₅ Wasserstoff ist oder zusammen mit R₅ₐ ein Oxo bildet;
R₅ₐ Wasserstoff, Hydroxy, geschütztes Hydroxy, Acyloxy ist, zusammen mit R₅ ein Oxo bildet oder zusammen mit R₄ und den Kohlenstoffatomen, an denen sie hängen, einen Oxetanring bildet;
R₆ Wasserstoff, Alkyl, Alkenyl, Alkynyl, Aryl oder Heteroaryl, Hydroxy, geschütztes Hydroxy ist oder zusammen mit R₆ₐ ein Oxo bildet;
R₆ₐ Wasserstoff, Alkyl, Alkenyl, Alkynyl, Aryl oder Heteroaryl, Hydroxy, geschütztes Hydroxy ist oder zusammen mit R₆ ein Oxo bildet;
R₇ Wasserstoff ist oder zusammen mit R₇ₐ ein Oxo bildet;
R₇ₐ Wasserstoff, Halogen, geschütztes Hydroxy, -OR₂₈ ist oder zusammen mit R₇ ein Oxo bildet;
R₉ Wasserstoff ist oder zusammen mit R₉ₐ ein Oxo bildet;
R₉ₐ Wasserstoff, Hydroxy, geschütztes Hydroxy, Acyloxy ist oder zusammen mit R₉ ein Oxo bildet;
R₁₀ Wasserstoff ist oder zusammen mit R₁₀ₐ ein Oxo bildet;
R₁₀ₐ Wasserstoff, -OCOR₂₉ , Hydroxy oder geschütztes Hydroxy ist oder zusammen mit R₁₀ ein Oxo bildet;
R₁₄ Wasserstoff, Hydroxy, geschütztes Hydroxy ist oder zusammen mit R₁ ein Carbonat bildet;
R₁₄ₐ Wasserstoff ist;
R₂₈ Wasserstoff, Acyl oder eine Hydroxy-Schutzgruppe ist; und
R₂₉ , R₃₀ und R₃₁ unabhängig Wasserstoff, Alkyl, Alkenyl, Alkynyl, monocyclisches Aryl oder monocyclisches Heteroaryl sind.

10. Taxanderivat des Anspruchs 9, bei dem X₅ -COX₁₀ ist, worin X₁₀ Furyl, Thienyl, alkylsubstituiertes Furyl oder Thienyl ist oder X₅ ist -COOX₁₀ , worin X₁₀ t-Butyl, iso-Butyl oder n-Butyl, Ethyl, iso-Propyl, n-Propyl, Cyclohexyl, Allyl, Crotyl, 1,3-Diethoxy-2-propyl, 2-Methoxyethyl, Amyl, Neopentyl oder PhCH₂- ist, oder X₅ ist -CONX₈X₁₀ , worin X₈ und X₁₀ beide Phenyl sind oder X₈ Wasserstoff ist und X₁₀ n-Propyl, Phenyl oder Ethyl ist.

11. β-Lactam der Formel 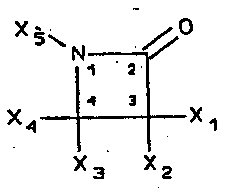 worin
X₁ -OX₆ , -SX₇ oder -NX₈ X₉ ist;
X₂ Wasserstoff, Alkyl, Alkenyl, Alkynyl, Aryl oder Heteroaryl ist;
X₃ Wasserstoff ist;
X₄ Isobutenyl ist;
X₅ -COX₁₃ , -COOX₁₀ , -COSX₁₂ , -CONX₈X₁₂ oder -SO₂X₁₁ ist;
X₆ Wasserstoff, Alkyl, Alkenyl, Alkynyl, Aryl oder Heteroaryl ist;
X₇ Alkyl, Alkenyl, Alkynyl, Aryl, Heteroaryl oder eine Sulfhydryl-Schutzgruppe ist;
X₈ Wasserstoff, Alkyl, Alkenyl, Alkynyl, Aryl, Heteroaryl oder heterosubstituiertes Alkyl, Alkenyl, Alkynyl, Aryl oder Heteroaryl ist;
X₉ eine Amino-Schutzgruppe ist;
X₁₀ Alkenyl, Alkynyl, Aryl, Heteroaryl oder heterosubstituiertes Alkyl, Alkenyl, Alkynyl Aryl oder Heteroaryl ist;
X₁₁ Alkyl, Alkenyl, Alkynyl, Aryl, Heteroaryl, -OX₁₀ oder -NX₈X₁₄ ist;
X₁₂ Alkyl, Alkenyl, Alkynyl, Aryl, Heteroaryl oder heterosubstituiertes Alkyl, Alkenyl, Alkynyl, Aryl oder Heteroaryl ist;
X₁₃ Alkenyl, Alkynyl, Heteroaryl oder heterosubstituiertes Alkyl, Alkenyl, Alkynyl oder Heteroaryl ist;
X₁₄ Wasserstoff, Alkyl, Alkenyl, Alkynyl, Aryl oder Heteroaryl ist.

12. β-Lactam der Formel 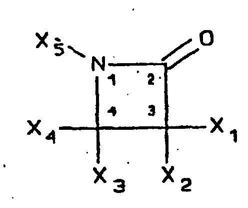 worin
X₁ -OX₆ , -SX₇ oder -NX₈X₉ist;
X₂ Alkenyl, Alkynyl, Aryl oder Heteroaryl ist;
X₃ Wasserstoff ist;
X₄ Isobutenyl ist;
X₅ -COX₁₀ , -COOX₁₀ , -COSX₁₀ , -CONX₈X₁₀ oder -SO₂X₁₁ ist;
X₆ Wasserstoff, Alkyl, Alkenyl, Alkynyl, Aryl oder Heteroaryl ist;
X₇ Alkyl, Alkenyl, Alkynyl, Aryl, Heteroaryl oder eine Sulfhydryl-Schutzgruppe ist;
X₈ Wasserstoff, Alkyl, Alkenyl, Alkynyl, Aryl, Heteroaryl oder heterosubstituiertes Alkyl, Alkenyl, Alkynyl, Aryl oder Heteroaryl ist;
X₉ eine Amino-Schutzgruppe ist;
X₁₀ Alkyl, Alkenyl, Alkynyl, Aryl, Heteroaryl oder heterosubstituiertes Alkyl, Alkenyl, Alkynyl, Aryl oder Heteroaryl ist;
X₁₁ Alkyl, Alkenyl, Alkynyl, Aryl, Heteroaryl, -OX₁₀ oder -NX₈X₁₀ ist;
X₁₄ Wasserstoff, Alkyl, Alkenyl, Alkynyl, Aryl oder Heteroaryl ist.

## Revendications

1. Dérivé de taxane de formule 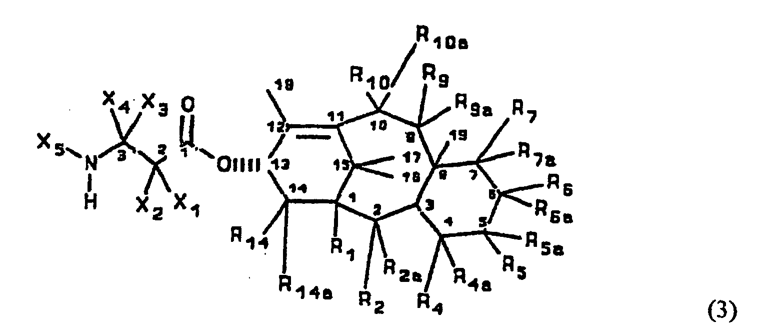 dans laquelle
X₁ est un groupe -OX₆, -SX₇ ou -NX₈X₉;
X₂ est un atome d'hydrogène ou un groupe alkyle, alcényle, alcynyle, aryle ou hétéroaryle;
X₃ est un atome d'hydrogène;
X₄ est un groupe isobutényle;
X₅ est un groupe -COX₁₀, -COOX₁₀, -COSX₁₀, -CONX₈X₁₀ ou -SO₂X₁₁;
X₆ est un atome d'hydrogène ou un groupe alkyle, alcényle, alcynyle, aryle ou hétéroaryle;
X₇ est un groupe alkyle, alcényle, alcynyle, aryle ou hétéroaryle ou un groupe protecteur de sulfhydryle;
X₈ est un atome d'hydrogène, un groupe alkyle, alcényle, alcynyle, aryle ou hétéroaryle ou un groupe alkyle, alcényle, alcynyle, aryle ou hétéroaryle hétérosubstitué;
X₉ est un groupe protecteur d'amino;
X₁₀ est un groupe alkyle, alcényle, alcynyle, aryle ou hétéroaryle ou un groupe alkyle, alcényle, alcynyle, aryle ou hétéroaryle hétérosubstitué;
X₁₁ est un groupe alkyle, alcényle, alcynyle, aryle, hétéroaryle, -OX₁₀ ou -NX₈X₁₄;
X₁₄ est un atome d'hydrogène ou un groupe alkyle, alcényle, alcynyle, aryle ou hétéroaryle;
R₁ est un atome d'hydrogène ou un groupe hydroxy ou hydroxy protégé ou forme un carbonate avec R₁₄;
R₂ est un atome d'hydrogène ou un groupe hydroxy ou -OCOR₃₁, ou forme un groupe oxo avec R₂ₐ;
R₂ₐ est un atome d'hydrogène ou, pris ensemble avec R₂, forme un groupe oxo;
R₄ est un atome d'hydrogène ou forme un groupe oxo, oxirane ou méthylène avec R₄ₐ, ou forme un cycle oxétane avec R₅ₐ et les atomes de carbone auxquels ils sont liés;
R₄ₐ est un atome d'hydrogène ou un groupe alkyle, alcényle, alcynyle, aryle, hétéroaryle, cyano, hydroxy, -OCOR₃₀, ou forme avec R₄ un groupe oxo, oxirane ou méthylène;
R₅ est un atome d'hydrogène ou forme un groupe oxo avec R₅ₐ;
R₅ₐ est un atome d'hydrogène ou un groupe hydroxy, hydroxy protégé ou acyloxy, ou forme un groupe oxo avec R₅ ou forme un cycle oxétane avec R₄ et les atomes de carbones auxquels ils sont liés;
R₆ est un atome d'hydrogène ou un groupe alkyle, alcényle, alcynyle, aryle, hétéroaryle, hydroxy ou hydroxy protégé, ou forme un groupe oxo avec R₆ₐ;
R₆ₐ est un atome d'hydrogène, un groupe alkyle, alcényle, alcynyle, aryle, hétéroaryle, hydroxy ou hydroxy protégé, ou forme un groupe oxo avec R₆;
R₇ est un atome d'hydrogène ou forme un groupe oxo avec R₇ₐ;
R₇ₐ est un atome d'hydrogène ou d'halogène, un groupe hydroxy protégé ou -OR₂₈, ou forme un groupe oxo avec R₇;
R₉ est un atome d'hydrogène ou forme un groupe oxo avec R₉ₐ;
R₉ₐ est un atome d'hydrogène, un groupe hydroxy, hydroxy protégé ou acyloxy, ou forme un groupe oxo avec R₉;
R₁₀ est un atome d'hydrogène ou forme un groupe oxo avec R₁₀ₐ;
R₁₀ₐ est un atome d'hydrogène, un groupe -OCOR₂₉, hydroxy ou hydroxy protégé, ou forme un groupe oxo avec R₁₀;
R₁₄ est un atome d'hydrogène, un groupe alkyle, alcényle, alcynyle, aryle, hétéroaryle, hydroxy ou hydroxy protégé ou forme un carbonate avec R₁;
R₁₄ₐ est un atome d'hydrogène ou un groupe alkyle, alcényle, alcynyle, aryle ou hétéroaryle;
R₂₈ est un atome d'hydrogène, un groupe acyle ou un groupe protecteur d'hydroxy; et
R₂₉, R₃₀ et R₃₁ sont indépendamment un atome d'hydrogène ou un groupe alkyle, alcényle, alcynyle, aryle monocyclique ou hétéroaryle monocyclique.

2. Dérivé de taxane selon la revendication 1, dans lequel X₅ est un groupe -COOX₁₀ et X₁₀ est un groupe alcoxy.

3. Dérivé de taxane selon la revendication 1, dans lequel
R₁₄ et R₁₄ₐ sont des atomes d'hydrogène;
R₁₀ est un atome d'hydrogène ou forme un groupe oxo avec R₁₀ₐ;
R₁₀ₐ est un atome d'hydrogène, un groupe hydroxy ou -OCOR₂₉ ou forme un groupe oxo avec R₁₀;
R₉ est un atome d'hydrogène ou forme un groupe oxo avec R₉ₐ;
R₉ₐ est un atome d'hydrogène, un groupe acyloxy, ou forme un groupe oxo avec R₉;
R₇ est un atome d'hydrogène;
R₇ₐ est un atome d'hydrogène ou d'halogène ou un groupe -OR₂₈;
R₆ et R₆ₐ sont des atomes d'hydrogène;
R₅ est un atome d'hydrogène;
R₅ₐ forme un cycle oxétane avec R₄ et les atomes de carbone auxquels ils sont liés;
R₄ₐ est un groupe hydroxy ou -OCOR₃₀;
R₂ est un groupe hydroxy ou -OCOR₃₁;
R₂ₐ est un atome d'hydrogène;
R₁ est un atome d'hydrogène ou un groupe hydroxy;
R₂₈ est un atome d'hydrogène, un groupe acyle ou un groupe protecteur d'hydroxy;
R₂₉, R₃₀ et R₃₁ sont indépendamment un atome d'hydrogène ou un groupe alkyle, alcényle, alcynyle, aryle monocyclique ou hétéroaryle monocyclique.

4. Dérivé de taxane selon la revendication 2, dans lequel
R₁₄ et R₁₄ₐ sont des atomes d'hydrogène;
R₁₀ est un atome d'hydrogène ou forme un groupe oxo avec R₁₀ₐ;
R₁₀ₐ est un atome d'hydrogène, un groupe hydroxy ou -OCOR₂₉ ou forme un groupe oxo avec R₁₀;
R₉ est un atome d'hydrogène ou forme un groupe oxo avec R₉ₐ;
R₉ₐ est un atome d'hydrogène, un groupe acyloxy, ou forme un groupe oxo avec R₉;
R₇ est un atome d'hydrogène;
R₇ₐ est un atome d'hydrogène ou d'halogène ou un groupe -OR₂₈;
R₆ et R₆ₐ sont des atomes d'hydrogène;
R₅ est un atome d'hydrogène;
R₅ₐ forme un cycle oxétane avec R₄ et les atomes de carbone auxquels ils sont liés;
R₄ₐ est un groupe hydroxy ou -OCOR₃₀;
R₂ est un groupe hydroxy ou -OCOR₃₁;
R₂ₐ est un atome d'hydrogène;
R₁ est un atome d'hydrogène ou un groupe hydroxy;
R₂₈ est un atome d'hydrogène, un groupe acyle ou un groupe protecteur d'hydroxy;
R₂₉, R₃₀ et R₃₁ sont indépendamment un atome d'hydrogène ou un groupe alkyle, alcényle, alcynyle, aryle monocyclique ou hétéroaryle monocyclique.

5. Taxane selon la revendication 1, dans lequel R₁₄ et R₁₄ₐ sont des atomes d'hydrogène, R₁₀ est un atome d'hydrogène, R₁₀ₐ est un groupe hydroxy ou acétoxy, R₉ et R₉ₐ forment ensemble un groupe oxo, R₇ est un atome d'hydrogène, R₇ₐ est un groupe hydroxy, R₅ est un atome d'hydrogène, R₅ₐ et R₄ et les atomes de carbone auxquels ils sont liés forment un cycle oxétane, R₄ₐ est un groupe acétoxy, R₂ est un atome d'hydrogène, R₂ₐ est un groupe benzoyloxy et R₁ est un groupe hydroxy.

6. Taxane selon la revendication 1, dans lequel R₁₄ est un groupe hydroxy, R₂ est un groupe hydroxy ou -OCOR₃₁ dans lequel R₃₁ est un atome d'hydrogène ou un groupe alkyle ou est choisi dans le groupe constitué par 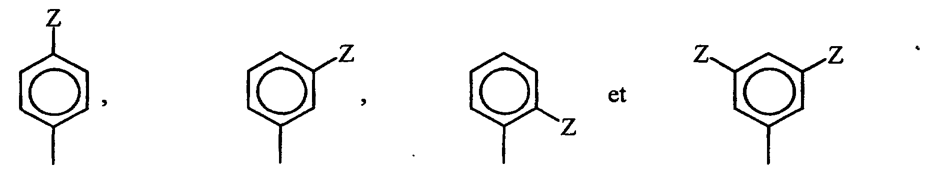 et Z est un groupe alkyle, hydroxy, alcoxy, halogéno ou trifluorométhyle.

7. Taxane selon la revendication 1, dans lequel R₉ₐ est un atome d'hydrogène et R₉ est un atome d'hydrogène ou un groupe hydroxy, R₇ₐ est un atome d'hydrogène et R₇ est un groupe acétoxy ou un autre groupe acyloxy ou un halogène, ou R₁₀ et R₁₀ₐ sont chacun un atome d'hydrogène ou forment ensemble un groupe oxo.

8. Taxane selon la revendication 1, dans lequel X₁ est un groupe -OH, X₂ est un atome d'hydrogène, X₃ est un atome d'hydrogène, X₄ est un groupe isobutényle, X₅ est un groupe -COX₁₀ dans lequel X₁₀ est un groupe furyle ou thiényle ou un groupe furyle ou thiényle substitué par alkyle, ou X₅ est un groupe -COOX₁₀ dans lequel X₁₀ est un groupe t-butyle, isobutyle, n-butyle, éthyle, isopropyle, n-propyle, cyclohexyle, allyle, crotyle, 1,3-diéthoxy-2-propyle, 2-méthoxyéthyle, amyle, néopentyle ou PhCH₂-, ou X₅ est un groupe -NX₈X₁₀ dans lequel X₈ et X₁₀ sont tous les deux un groupe phényle, ou X₈ est un atome d'hydrogène et X₁₀ est un groupe n-propyle, phényle ou éthyle.

9. Dérivé de taxane de formule 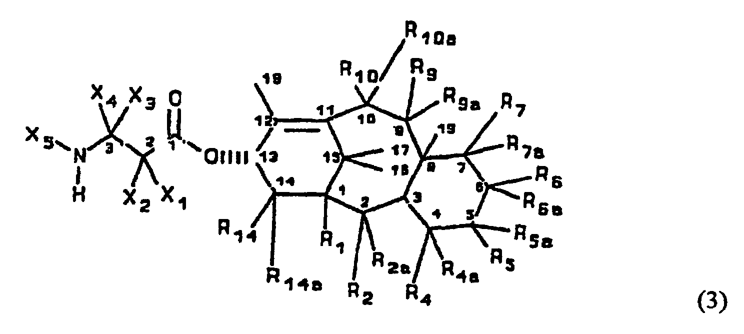 dans laquelle
X₁ est un groupe -OX₆;
X₂ est un atome d'hydrogène;
X₃ est un atome d'hydrogène;
X₄ est un groupe isobutényle;
X₅ est un groupe -COX₁₀, -COOX₁₀, -COSX₁₀, -CONX₈X₁₀ ou -SO₂X₁₁;
X₆ est un atome d'hydrogène;
X₈ est un atome d'hydrogène, un groupe alkyle, alcényle, alcynyle, aryle ou hétéroaryle ou un groupe alkyle, alcényle, alcynyle, aryle ou hétéroaryle hétérosubstitué;
X₉ est un groupe protecteur d'amino;
X₁₀ est un groupe alkyle, alcényle, alcynyle, aryle ou hétéroaryle ou un groupe alkyle, alcényle, alcynyle, aryle ou hétéroaryle hétérosubstitué;
X₁₁ est un groupe alkyle, alcényle, alcynyle, aryle, hétéroaryle, -OX₁₀ ou -NX₈X₁₄;
X₁₄ est un atome d'hydrogène ou un groupe alkyle, alcényle, alcynyle, aryle ou hétéroaryle;
R₁ est un atome d'hydrogène ou un groupe hydroxy ou hydroxy protégé ou forme un carbonate avec R₁₄;
R₂ est un atome d'hydrogène ou un groupe hydroxy ou -OCOR₃₁, ou forme un groupe oxo avec R₂ₐ;
R₂ₐ est un atome d'hydrogène ou, pris ensemble avec R₂, forme un groupe oxo;
R₄ est un atome d'hydrogène ou forme un groupe oxo, oxirane ou méthylène avec R₄ₐ, ou forme un cycle oxétane avec R₅ₐ et les atomes de carbone auxquels ils sont liés;
R₄ₐ est un atome d'hydrogène ou un groupe alkyle, alcényle, alcynyle, aryle, hétéroaryle, cyano, hydroxy, -OCOR₃₀, ou forme avec R₄ un groupe oxo, oxirane ou méthylène;
R₅ est un atome d'hydrogène ou forme un groupe oxo avec R₅ₐ;
R₅ₐ est un atome d'hydrogène ou un groupe hydroxy, hydroxy protégé ou acyloxy, ou forme un groupe oxo avec R₅ ou forme un cycle oxétane avec R₄ et les atomes de carbones auxquels ils sont liés;
R₆ est un atome d'hydrogène ou un groupe alkyle, alcényle, alcynyle, aryle, hétéroaryle, hydroxy ou hydroxy protégé, ou forme un groupe oxo avec R₆ₐ;
R₆ₐ est un atome d'hydrogène, un groupe alkyle, alcényle, alcynyle, aryle, hétéroaryle, hydroxy ou hydroxy protégé, ou forme un groupe oxo avec R₆;
R₇ est un atome d'hydrogène ou forme un groupe oxo avec R₇ₐ;
R₇ₐ est un atome d'hydrogène ou d'halogène, un groupe hydroxy protégé ou -OR₂₈, ou forme un groupe oxo avec R₇;
R₉ est un atome d'hydrogène ou forme un groupe oxo avec R₉ₐ;
R₉ₐ est un atome d'hydrogène, un groupe hydroxy, hydroxy protégé ou acyloxy, ou forme un groupe oxo avec R₉;
R₁₀ est un atome d'hydrogène ou forme un groupe oxo avec R₁₀ₐ;
R₁₀ₐ est un atome d'hydrogène, un groupe -OCOR₂₉, hydroxy ou hydroxy protégé, ou forme un groupe oxo avec R₁₀;
R₁₄ est un atome d'hydrogène, un groupe hydroxy ou hydroxy protégé ou forme un carbonate avec R₁;
R₁₄ₐ est un atome d'hydrogène;
R₂₈ est un atome d'hydrogène, un groupe acyle ou un groupe protecteur d'hydroxy; et
R₂₉, R₃₀ et R₃₁ sont indépendamment un atome d'hydrogène ou un groupe alkyle, alcényle, alcynyle, aryle monocyclique ou hétéroaryle monocyclique.

10. Dérivé de taxane selon la revendication 9, dans lequel X₅ est un groupe -COX₁₀ dans lequel X₁₀ est un groupe furyle ou thiényle ou un groupe furyle ou thiényle substitué par alkyle, ou X₅ est un groupe -COOX₁₀ dans lequel X₁₀ est un groupe t-butyle, isobutyle, n-butyle, éthyle, isopropyle, n-propyle, cyclohexyle, allyle, crotyle, 1,3-diéthoxy-2-propyle, 2-méthoxyéthyle, amyle, néopentyle ou PhCH₂-, ou X₅ est un groupe -CONX₈X₁₀ dans lequel X₈ et X₁₀ sont tous les deux un groupe phényle, ou X₈ est un atome d'hydrogène et X₁₀ est un groupe n-propyle, phényle ou éthyle.

11. β-lactame de formule 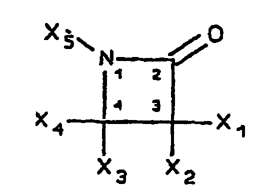 dans laquelle
X₁ est un groupe -OX₆, -SX₇ ou -NX₈X₉;
X₂ est un atome d'hydrogène ou un groupe alkyle, alcényle, alcynyle, aryle ou hétéroaryle;
X₃ est un atome d'hydrogène;
X₄ est un groupe isobutényle;
X₅ est un groupe -COX₁₃, -COOX₁₀, -COSX₁₂, -CONX₈X₁₂ ou -SO₂X₁₁;
X₆ est un atome d'hydrogène ou un groupe alkyle, alcényle, alcynyle, aryle ou hétéroaryle;
X₇ est un groupe alkyle, alcényle, alcynyle, aryle ou hétéroaryle ou un groupe protecteur de sulfhydryle;
X₈ est un atome d'hydrogène, un groupe alkyle, alcényle, alcynyle, aryle ou hétéroaryle ou un groupe alkyle, alcényle, alcynyle, aryle ou hétéroaryle hétérosubstitué;
X₉ est un groupe protecteur d'amino;
X₁₀ est un groupe alcényle, alcynyle, aryle, ou hétéroaryle ou un groupe alkyle, alcényle, alcynyle, aryle ou hétéroaryle hétérosubstitué;
X₁₁ est un groupe alkyle, alcényle, alcynyle, aryle, hétéroaryle, -OX₁₀ ou -NX₈X₁₄;
X₁₂ est un groupe alkyle, alcényle, alcynyle, aryle ou hétéroaryle ou un groupe alkyle, alcényle, alcynyle, aryle ou hétéroaryle hétérosubstitué;
X₁₃ est un groupe alcényle, alcynyle ou hétéroaryle ou un groupe alkyle, alcényle, alcynyle ou hétéroaryle hétérosubstitué;
X₁₄ est un atome d'hydrogène ou un groupe alkyle, alcényle, alcynyle, aryle ou hétéroaryle.

12. β-lactame de formule 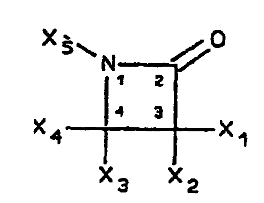 dans laquelle
X₁ est un groupe -OX₆, -SX₇ ou NX₈X₉;
X₂ est un groupe alcényle, alcynyle, aryle ou hétéroaryle;
X₃ est un atome d'hydrogène;
X₄ est un groupe isobutényle;
X₅ est un groupe -COX₁₀, -COOX₁₀, -COSX₁₀, -CONX₈X₁₀ ou -SO₂X₁₁;
X₆ est un atome d'hydrogène ou un groupe alkyle, alcényle, alcynyle, aryle ou hétéroaryle;
X₇ est un groupe alkyle, alcényle, alcynyle, aryle ou hétéroaryle ou un groupe protecteur de sulfhydryle;
X₈ est un atome d'hydrogène, un groupe alkyle, alcényle, alcynyle, aryle ou hétéroaryle ou un groupe alkyle, alcényle, alcynyle, aryle ou hétéroaryle hétérosubstitué;
X₉ est un groupe protecteur d'amino;
X₁₀ est un groupe alkyle, alcényle, alcynyle, aryle, ou hétéroaryle ou un groupe alkyle, alcényle, alcynyle, aryle ou hétéroaryle hétérosubstitué;
X₁₁ est un groupe alkyle, alcényle, alcynyle, aryle, hétéroaryle, -OX₁₀ ou -NX₈X₁₄;
X₁₄ est un atome d'hydrogène ou un groupe alkyle, alcényle, alcynyle, aryle ou hétéroaryle.
